# EUROPEAN PATENT SPECIFICATION

(11) **EP 0 835 097 B1**
(45) Date of publication and mention of the grant of the patent: **29.08.2001**
(21) Application number: 96917214.7
(22) Date of filing: 06.06.1996
(51) Int. Cl.: A61K 9/107

(54) **MIXED LIPID-BICARBONATE COLLOIDAL PARTICLES FOR DELIVERING DRUGS OR CALORIES**
GEMISCHTE LIPID-BICARBONAT KOLLOIDALE PARTIKEL ZUR ABGABE VON ARZNEISTOFFEN ODER KALORIEN
PARTICULES COLLOIDALES LIPIDIQUES MELANGEES ET DE BICARBONATE POUR L'ADMINISTRATION DE MEDICAMENTS OU DE CALORIES

(30) Priority: 06.06.1995 US 470742
(43) Date of publication of application: 15.04.1998
(73) Proprietor: Biomolecular Products, Inc., Byfield, MA 01922 (US)
(72) Inventor: YESAIR, David, W., Byfield, MA 01922 (US)
(74) Representative: Killin, Stephen James
(86) International application number: US9609031
(87) International publication number: WO9639120

(56) References cited:
- WO-A-86/05694
- WO-A-92/03121

## Description

Drugs must reach their targets selectively and controllably if their desired pharmacological activities are to be maximized. One approach to optimizing the activities of drugs is to control and sustain their delivery into the systemic blood circulation. Orally administered drugs are generally absorbed in the intestine. Such drugs undergo first pass clearance by the liver and small intestine; that is, they are converted by the intestine and the liver to pharmacologically inactive metabolites and/or are secreted into bile by the liver, either as drug or as active metabolites. As a result, the amount of an orally administered drug actually entering the systemic circulation can be much less than the amount administered. To ensure that effective quantities of such a drug will enter the circulation and reach the targeted site(s) in the body, larger quantities than actually needed must be administered and often must be given in several smaller doses, rather than one dose. Orally administered drugs also typically have poor bioavailability. For example, they may be adversely affected by the pH and the enzymatic activity of the stomach and intestine and may be poorly dissolved in the stomach and intestinal fluids.

There have been numerous attempts to address these problems and to improve the bioavailability of orally administered drugs. The efficacy of some drugs given orally has been improved by administering them with a triglyceride or neutral fat. Such fats represent an environment that is compatible with lipophilic drugs, i.e. that exhibit low aqueous solubility. Fats also enhance the stability of drugs which are unstable in the stomach and intestine. The end products of fat digestion are absorbed by the villi of the intestinal mucosa into a lymphatic vessel, the central lacteal; absorption occurs within a region of the intestine in which limited drug metabolism occurs. The absorbed fat is transported through the thoracic duct, the major lymphatic channel and is subsequently emptied into the blood; it is not carried in the portal blood, which goes to the liver, where first pass metabolism of drugs occurs.

The absorption of griseofulvin has been shown to be enhanced if the drug is coadministered with a high fat content meal or in an oil and water emulsion. Crounse, R.G., Journal of Investigative Dermatology, 37:529 (1961); Carrigan, P.J. and Bates, T.R., Journal of Pharmacological ,Science, 62:1476 (1973). If the hormone testosterone undecanoate is administered in a peanut oil solution, it is more biologically active than if it is administered in an aqueous microcrystalline suspension. Coert, A.J. et al., Acta Endocrinol, 79:789 (1975); Hirschhauser, C. et al., Acta Endocrinol, 80:179 (1975). This effect is presumed to be due to absorption of the steroid via the thoracic lymph rather than the portal blood; in this way, first pass clearance by the liver is avoided.

Cholesterol, its esters as well as triglyceride constituents (e.g., fatty acids and monoglycerides) are absorbed via the thoracic lymph. The effects of some of these compounds, alone or in the presence of bile salts, upon absorption of some orally administered drugs have been evaluated. For example, oral administration of ubidecarenone, which is used for treating hypertension, in a mixture containing fatty acids having 12-18 carbon atoms and monoglycerides containing such fatty acids, resulted in somewhat greater absorption of the ubidecarenone than occurred after oral administration of the drug alone (8.3% v. 2.3%). Taki, K. and Takahira, H., U.S. Pat. No. 4,325,942 (1982). If the steroid progesterone is administered orally in combination with cholesterol or its esters, good sustained biological activity can be obtained. This is believed to be due to the absorption of progesterone via the thoracic lymph and not via the portal circulation. Kind, F.A., Proceedings of the 6th International Congress of Pharmacology, 5:105 (1975).

Yesair has evaluated the effect of fatty acids having 12-18 carbon atoms, monoglycerides of these fatty acids, and bile salts on the absorption of orally administered estradiol, which is an estrogenic hormone. Yesair, D.W., PCT WO 83/00294 (1983). The mole ratio of fatty acids: monoglycerides:bile salts evaluated ranged from 10:1:1, 1:1:10 or 1:10:1. The preferred ratio was stated to be 4:1:4, which is similar to the micellar composition resulting from the enzymatic digestion of triglycerides in the intestine, which occurs in the presence of bile salts and calcium ions. When excess bile salts are present, estradiol incorporated into the 4:1:4 composition can migrate or partition into a bile salt-enriched micellar solution. This migration or partitioning of estradiol occurred prior to absorption of the drug, as shown by the fact that the initial concentrations in plasma of estradiol are initially greater than those in lymph. In addition, about 25-50% of the estradiol administered in the composition was co-absorbed with the lipid constituents and entered the systemic circulation via the thoracic lymph. The presence of bile salts, which are absorbed in the ileum (and not in the jejunum, as is most fat) compromised the co-absorption of estradiol with fat by enhancing the migration of the drug from fat to the bile salt micelle. Phosphatidylcholine was used in an effort to maintain the estradiol within the micellar composition in which fatty acids: monoglycerides:bile salts occurred in a 4:1:4 molar ratio. In the presence of excess bile salts, about 60% of the estradiol incorporated into the 4:1:4 micellar composition remained associated with it when phosphatidylcholine was not present. Under the same conditions, about 70-75% of the estradiol remained in the composition when phosphatidylcholine was used. Addition of phosphatidylcholine for this purpose, however, results in an increased size of the delivery system. Size is an important parameter in the absorption of lipid micelles and this effect of phosphatidylcholine might interfere with co-absorption of the drug with the lipids. In addition, excess phosphatidylcholine has been shown to reduce lipid absorption. Ammon, H.V., et al., Lipids, 14:395 (1979); Clark, S. B., Gastrointestinal Physiology, 4:E183 (1978).

Others have also described the effects of the presence of bile salts in lipid formulations used for co-absorption of drugs. Wilson, T.H., In: Intestinal Absorption. Saunders, (1962); Lack, L. and Weiner, I.M., American Journal of Physiology, 240:313, (1961); H.V. Ammon et al., Lipids, 14:395 (1979). For example, little difference in the absorption of 5-fluorouracil (5FU) in the stomach or small intestine was evident when the 5FU was administered alone or in a monoolein/sodium taurocholate mixed micelle formulation. 5FU absorption in the large intestine was greater when the drug was administered in the formulation than when it was administered alone. Streptomycin is poorly absorbed from the intestine. Muranushi and co-workers report that mixed micelles, composed of bile salts, monoolein or unsaturated fatty acids, did not improve the absorption of streptomycin from the small intestine but markedly enhanced the absorption from the large intestine. The enhancement in the large intestine was attributed mostly to the alteration of the mucosal membrane permeability by monoolein or unsaturated fatty acids. In contrast, mixed micelles of bile salts and saturated fatty acids produced only a small enhancement in streptomycin absorption even from the large intestine. Muranushi, N. et al., Journal of Pharmaceutics, 4:271 (1980). Taniguchi et al. report that mono-olein/taurocholate or oleic acid/taurocholate promotes the absorption of heparin, which is poorly absorbed when administered alone. Taniguchi, K. et al., International Journal of Pharmaceutics, 4:219 (1980). Absorption of heparin from the large intestine was twice that which occurred from the small intestine. The concentration of heparin in the mixed micelle to produce the potentiation in the large intestine was approximately one-fourth that required in the small intestine.

In U.S. Pat. No. 4,156,719, Sezoski and Muranishi describe a micelle solution for rectal administration of water-soluble drugs that are poorly absorbed. The composition consists of fatty acids having 6-18 carbons, and/or mono- or diglycerides having the same type of fatty acids; a bile salt or other non-ionic surface activity agent; and water. A lysophosphatidylcholine moiety can be substituted for the fatty acids and mono- or diglycerides. Absorption of streptomycin and gentamycin from the rectum and large intestine is reported to be comparable when the drug is administered in a bile salt:mixed lipid micelle. Similar formulations were not effective in increasing absorption in the duodenum. Muranushi, S. et al., International Journal of Pharmaceutics, 2:101 (1979). Absorption of the two drugs via the rectum and large intestine was markedly greater than that of a comparable dose administered duodenally, even when the mixed lipid micelle concentration administered duodenally was four times that administered via the other routes. In a patent to the present inventor (U.S. Patent No. 4,874,795, Yesair) it was shown that a lipid composition with specific lipid components in a prescribed relationship to each other was effective in delivering drugs to the systemic circulation. The lipid composition included fatty acids having 14-18 carbon atoms, monoglycerides with a fatty acid moiety having 14-18 carbon atoms, and lysophosphatidycholine with a fatty acid moiety having 14-18 carbon atoms. The fatty acid to monoglyceride molar ratio could range from 4:1 to 1:4 and the mole percent of lysophosphatidylcholine could range from 30.0 to 1.0 when expressed as the mole percent of the total lipid composition. This lipid composition was shown to effectively transport drugs to the systemic circulation when they were incorporated into the lipid composition. The lipid composition also was shown to serve as a source of calories by virtue of its inherent fatty acid content that could be metabolized in an individual's body.

Caloric requirements for individuals are primarily a function of body composition and level of physical activity. Medically compromised, e.g. cystic fibrosis patients, as well as aged and physically stressed individuals often have limited body fat. Consequently, energy (caloric) needs for these individuals will be satisfied mainly from exogenous sources.

Physical activity uses muscle and the energy requirements of all muscles, including the heart, are met primarily as a result of oxidation of fatty acids, from dietary fat or mobilized adipose fat. Adipose fat can, as noted, be minimal and therefore efficient absorption of fat can be an important consideration in satisfying the energy demands of the medically infirm, the aged and the physically active.

Fat absorption can be compromised in many circumstances. For example, in cystic fibrosis, a disorder of exocrine glands, there is a deficiency of pancreatic enzymes, bile salts and bicarbonate ions. Nutrition Reviews, 42:344 (1984); Ross, C.A., Archives of Diseases of Childhood, 30:316 (1955); Scow, R.O.E., Journal of Clinical Investigation, 55:908 (1975). Fat absorption in cystic fibrosis patients can be severely affected and 30 to 60 percent of ingested fat can be malabsorbed. The malabsorption and resulting steatorrhea are generally not successfully handled by the oral administration of pancreatic lipase. In an effort to control the steatorrhea, the patient may consume less fat than desirable for good health.

Compromised fat absorption can also reduce the exogenous intake of polyunsaturated fatty acids and choline, both of which are required for good health.

Fat absorption can be compromised under stressful conditions and the generally accepted way of addressing this problem has been to reduce fat consumption. This approach can result in both acute and chronic medical problems. These problems might be avoided, or at least minimized, if a readily absorbable source of fat could be made available.

At the present time, there is a need for a more efficient method of transporting orally administered drugs to the systemic circulation. This need is particularly important for individuals with impaired oral intake, intestinal absorption or diminished transport capacity. At - the same time, there is a need for a more efficient oral administration of calorically rich substances, especially to individuals with acute energy requirements. The achievement of such increased efficiencies would promote more effective drug therapies and nutritional stability.

The invention provides improved mixed lipid colloidal particles for the sustained delivery of bioactive agents. The invention also provides such compositions for use in delivering a bioactive agent to an individual in need of such a bioactive agent. Further, the invention provides pharmaceutical preparations including mixed lipid colloidal particle and a pharmaceutically acceptable carrier. The invention also provides for the use of such colloidal particles in the manufacture of a medicament. The invention also provides for the use of colloidal particles in the manufacture of a medicament for treatment of certain conditions.

According to one aspect of the invention, a composition of mixed particle size is provided. The composition can be for sustained delivery of a bioactive agent to an individual is provided. The composition includes a mixture of colloidal particles in an aqueous environment. Each colloidal particle of the mixture contains at least one non-esterified fatty acid having 8-24 carbon atoms, at least one monoglyceride which is a monoester of glycerol and a fatty acid having 8-24 carbon atoms, lysophosphatidylcholine in which the fatty acid moiety has 8-24 carbon atoms, and a titrating agent. The fatty acids and monoglycerides together comprise from about 70.0 mole percent to about 99.0 mole percent of the lipid composition. The molar ratio of the fatty acids to the monoglycerides is from about 4:1 to about 1:4, and the lysophosphatidylcholine comprises from about 30.0 mole percent to about 1.0 mole percent of the lipid composition. The lipid composition is in the form of colloidal particles in an aqueous environment. The mixture of colloidal particles is prepared from at least two separately prepared sets of colloidal particles of different size distributions. The first and second sets of colloidal particles differ in particle size in that at least 80% of the particle size distribution of the first set of particles is less than the average particle size of the second set of colloidal particles, and at least 80% of the particle size distribution of the second set of colloidal particles is greater than the average particle size of the first set of colloidal particles.

In some embodiments, the first set of colloidal particles has a molar ratio of the titrating agent to the lysophosphatidylcholine of less than 1.4:1, and the second set of colloidal particles has a molar ratio of titrating agent to the lysophosphatidylcholine between greater than 1.4:1 and about 10:1. Preferably, the second set of colloidal particles has a molar ratio of the tritating agent to the lysophosphatidylcholine between 1.4:1 and about 7:1. Most preferably, the titrating agent is bicarbonate.

In some embodiments, the fatty acids of the compositions include both omega-6 fatty acids and omega-3 fatty acids. Preferably the molar ratio of the omega-6 fatty acids to the omega-3 fatty acids is about 5:1. In certain embodiments, the omega-6 fatty acids and the omega-3 fatty acids comprise at least 50% of the total fatty acids in the composition.

In certain embodiments, the colloidal particles also include at least one biologically compatible surfactant. In preferred embodiments, the second set of colloidal particles has a molar ratio of the titrating agent to the lysophosphatidylcholine of at least 7:1, and a molar ratio of the biologically compatible surfactant to the lysophosphatidylcholine of at least 10:1. Preferably, the at least one biologically compatible surfactant is a bile salt, especially sodium taurocholate.

In certain embodiments, the bioactive agent delivered by the colloidal particles of the invention is a drug. In other embodiments, the bioactive agent is a polyunsaturated fatty acid. The polyunsaturated fatty acid may be incorporated into the particles as one of the non-esterified fatty acids, one of the fatty acid moieties of the monoglycerides and/or one of the fatty acids of the lysophosphatidylchloline. The bioactive agent may also be fat, for example, as a source of calories and/or choline.

According to another aspect of the invention, a composition for delivery of a bioactive agent to an individual in need of such a bioactive agent is provided. The delivery involves the administration of one of the compositions disclosed above. In one important embodiment, the bioactive agent delivered by the composition comprises a readily absorbable source of calories. Accordingly, the bioactive agent may be administered by oral administration, inhalation administration and/or topical administration.

According to another aspect of the invention, a composition for treating a condition with a bioactive agent effective for treating the condition is provided. This involves administering to a subject in need of the treatment an amount of one of the above disclosed compositions effective for treating the condition.

According to still another aspect of the invention, a pharmaceutical preparation is provided. The pharmaceutical preparation comprises the composition of the invention disclosed above, in combination with a pharmaceutically acceptable carrier.

According to yet another aspect of the invention, the use of one of the above disclosed compositions in the manufacture of a medicament is provided. In one embodiment the medicament is for delivery of a bioactive agent. Preferably, the medicament is an oral medicament, and inhalable medicament and/or a topical medicament. In other embodiments, the use of one of the compositions disclosed above is for the manufacture of a medicament for treatment of the nutritional requirements of decreased brain choline, liver damage, pregnancy, lactation, total parenteral nutrition, strenuous physical exertion, and/or newborns is provided.

According to still another aspect of the invention, the use of a composition comprising colloidal particles is provided for the manufacture of a medicament for the treatment of the nutritional requirements of decreased brain choline, liver damage, pregnancy, lactation, total parenteral nutrition, strenuous physical exertion, and/or newborns. The composition includes at least one non-esterified fatty acid having 8-24 carbon atoms, at least one monoglyceride which is a monoester of glycerol and a fatty acid having 8-24 carbon atoms and lysophosphatidylcholine in which the fatty acid moiety has 8-24 carbon atoms. In such compositions, the fatty acids and monoglycerides together comprise from about 70.0 mole percent to about 99.0 mole percent of the lipid composition. The molar ratio of the fatty acids to the monoglycerides is from about 4:1 to about 1:4, and the lysophosphatidylcholine comprises from about 30.0 mole percent to about 1.0 mole percent of the lipid composition. The lipid composition is in the form of colloidal particles in an aqueous environment.

In preferred embodiments, the composition further comprises a titrating agent. Preferably, the concentration of the lysophosphatidylcholine is at least 0.1mm in the aqueous environment and the molar ratio of the titrating agent to the lysophosphatidylcholine is greater than 1:1 in the aqueous environment. More preferably, the molar ratio of the titrating agent to the lysophosphatidylcholine is greater than 1.4:1. In preferred embodiments, the molar ratio of the titrating agent to the lysophosphatidylcholine is greater than 7:1. In certain of these preferred embodiments, the titrating agent is bicarbonate.

In certain embodiments, the use of compositions including colloidal particles of a single average size and at least one biologically compatible surfactant is provided. The molar ratio of the at least one biologically compatible surfactant to the lysophosphatidylcholine in the aqueous environment is at least 10:1. In preferred embodiments, the at least one biologically compatible surfactant is a bile salt. More preferably, the bile salt is sodium taurocholate.

According to another aspect of the invention, the use of a composition in the manufacture of a medicament for the treatment of cystic fibrosis is provided. The composition includes at least one non-esterified fatty acid having 8-24 carbon atoms, at least one monoglyceride which is a monoester of glycerol and a fatty acid having 8-24 carbon atoms and lysophosphatidylcholine in which the fatty acid moiety has 8-24 carbon atoms. In such compositions, the fatty acids and monoglycerides together comprise from about 70.0 mole percent to about 99.0 mole percent of the lipid composition. The molar ratio of the fatty acids to the monoglycerides is from about 4:1 to about 1:4, and the lysophosphatidylcholine comprises from about 30.0 mole percent to about 1.0 mole percent of the lipid composition. The lipid composition is in the form of colloidal particles in an aqueous environment.

Figure 1 is a graph showing the relationship between the surface tension of the mixed lipid formulation and the molar concentration of the lysophosphatidylcholine (and mixed lipids) in the mixed lipid formulation.

Figure 2 is a graph showing the relationship between the particle size or surface tension of the mixed lipid formulation and the amount of sodium bicarbonate in the aqueous environment.

Figure 3 is a graph showing the relationship between the particle size of the mixed lipid formulation and the amount of bile salt, sodium taurocholate, in the aqueous environment.

Figure 4 is a graph showing the relationship between the particle size or surface tension of the mixed lipid formulation and the combination of bicarbonate and bile salt, sodium taurocholate.

Figure 5 is a graph showing the elution profiles from a Sepharose 4B column of the mixed lipid (fenretinamide) bicarbonate, mixed lipid (fenretinamide)-bicarbonate-bile salt and free fenretinamide.

Figure 6 is a graph showing the elution profiles from a Sepharose 4B column of the mixed lipid-bicarbonate formulation and the mixed lipid-bicarbonate-bile salt formulation containing diltiazem (Drug A) and hydrochlorothiazide (Drug B).

Figure 7 is a graph showing the time profile of fat absorption for cystic fibrosis (CF) patients given a eutectic matrix (EM) of lipids or a triglyceride (TG) control formulation, and for control individuals given a colloidal eutectic matrix (CEM) of lipids or a triglyceride (TG) control formulation.

Figure 8 is a graph showing the total amount (or area under the curve (AUC)) of fat absorption for cystic fibrosis (CF) patients given a eutectic matrix (EM) of lipids or a triglyceride (TG) control formulation, and for control individuals given a colloidal eutectic matrix (CEM) of lipids or a triglyceride (TG) control formulation.

Figure 9 is a graph showing the time profile of fat absorption for a control individual given a colloidal eutectic matrix (CEM), eutectic matrix (EM) or a triglyceride control formulation.

Figure 10 is a graph showing the time profile average of fat absorption for five control individuals given a colloidal eutectic matrix (CEM) of lipids or a eutectic matrix (EM) of lipids.

Figure 11 is a graph showing the total amount (or area under the curve (AUC)) average of fat absorption for 5 control individuals given a colloidal eutectic matrix (CEM) of lipids or a eutectic matrix of lipids (EM).

The compositions of the present invention are comprised of non-esterified fatty acids, monoglycerides of those fatty acids, lysophosphatidylcholine having those fatty acids as their fatty acid moiety, and a titrating agent. The selection of the components of the subject composition is based on the absorption and transport characteristics of the fatty acids, the contribution of lysophosphatidylcholine to solubilization of drugs in the lipid composition, the properties of a titrating agent such as bicarbonate that allow stable, submicron size lipid-containing particles to exist and to translocation of absorbed fat into the lymph (rather than into the portal circulation).

Absorption of saturated fatty acids has been shown to be inversely related to the number of carbon atoms in the fatty acid. For example, absorption of decanoic (10:0, which denotes chain length and degree of unsaturation) is almost quantitative. For lauric (12:0), it is more than 95%; for myristic (14:0), 80-90%; for palmitic (16:0), 65-70% and for stearic (18:0), 30-45%. Absorption of unsaturated fatty acids into lymph (e.g., linoleic 18:2) have been shown to be more rapid and to a greater extent than are saturated fatty acids. Taniguchi, K., International Journal of Pharmaceutics, 4:219 (1980).

Transport of absorbed fatty acids via the lymph (and not in the portal circulation) varies greatly. That is, a much larger percentage of absorbed mono- or polyunsaturated fatty acids has been shown to be carried in the lymph than is the case for saturated fatty acids. About 85% of unsaturated fatty acids has been shown to be carried in the lymph. Miura, S. et al., Keio Journal of Medicine, 28:121 (1979). The amount of these absorbed fatty acids being carried in the lymph is also inversely related to chain length: 68-80% for myristic; 85% for palmitic and stearic. If saturated fatty acids are included in the composition of this invention, they can be included as calcium salts or salts of another cation. This is true because the enzymatic hydrolysis of triglycerides, which releases saturated fatty acids, favors their calcium soap formation. Tak, Y.A. and Grigor, M.R., Biochimica Biophysica Acta, 531:257 (1978).

Translocation of absorbed fat into the lymph has been shown to require lysophosphatidylcholine. The rate, but not the magnitude, of the translocation of absorbed fat is apparently related to the fatty acid moiety of the lysophosphatidylcholine. For example, oleoyl lysophosphatidylcholine results in a 100% increase in triglyceride and phospholipid in lymphatic transported fat when compared with the effects of a lysophosphatidylcholine derived from a phosphatidylcholine composed mainly of saturated fatty acids (e.g., palmitic, C16:0; stearic, C18:0). Incorporating a mono- or polyunsaturated lysophosphatidylcholine into the compositions of this invention will enhance the translocation of the absorbed lipids and the co-absorbed drugs or other substances. In addition, lysophosphatidylcholine plays a role in the solubilization of some drugs (i.e., its presence enhances the solubility of the drugs in the compositions) as well as providing a source of choline.

Examples of unsaturated fatty acids which can be used in the composition of this invention are:

| | | |
|---|---|---|
| palmitoleic | C₁₆H₃₀O₂ | 16:1 |
| oleic | C₁₈H₃₄O₂ | 18:1 |
| linoleic | C₁₈H₃₂O₂ | 18:2 |
| linolenic | C₁₈H₃₀O₂ | 18:3 |
| arachidonic | C₂₀H₃₂O₂ | 20:4 |
| eicosapentanoic | C₂₀H₃₀O₂ | 20:5 |
| docosahexanoic | C₂₂H₃₂O₂ | 22:6 |

Examples of saturated fatty acids which can be used in the subject composition are:

| | | |
|---|---|---|
| octanoic | C₈H₁₆O₂ | 8:0 |
| decanoic lauric | C₁₀H₂₀O₂ C₁₂H₂₄O₂ | 10:0 12:0 |
| myristic | C₁₄H₂₈O₂ | 14:0 |
| palmitic | C₁₆H₃₂O₂ | 16:0 |
| stearic | C₁₈H₃₆O₂ | 18:0 |

The monounsaturated, polyunsaturated and saturated fatty acids can be present individually or in combination. That is, the fatty acid constituents of one or more of the lipid components (fatty acid, monoglyceride and lysophosphatidylcholine) can be identified or they can be a mixture of the mono-, polyunsaturated and/or saturated members of the preferred fatty acid families. By choosing the fatty acid constituents, the ratio of the omega-6 fatty acids to the omega-3 fatty acids can be predetermined. Preferably, the ratio is about 5:1. The percentage compositions of omega-6 fatty acids and omega-3 fatty acids can be regulated in a similar manner and preferably the omega-6 fatty acids and the omega-3 fatty acids are greater than about 50% of the total fatty acids in the composition.

The non-esterified fatty acids and monoglycerides are present in amounts which result in a molar ratio of from about 4:1 to about 1:4 (non-esterified fatty acid: monoglyceride).

In addition, the compositions have lysophosphatidylcholine, the fatty acid moiety of which has 8-24 carbon atoms and is preferably mono- or polyunsaturated. The fatty acid constituent of the lysophosphatidylcholine is preferably one of those listed above. The quantity of lysophosphatidylcholine in the composition is determined by the amount needed for enhanced solubilization of a drug to be administered in the composition and the amount needed for its role in translocation. In general, lysophosphatidylcholine comprises from about 1.0 mole percent to about 30.0 mole percent of the total composition. The fatty acids which comprise the compositions of this invention, whether as nonesterified fatty acids or as constituents of monoglycerides or lysophosphatidylcholine, can all be the same or a number of different ones can be included.

Lipid formulations including the fatty acids, monoglycerides and lysophosphatidylcholine described above will swell in the presence of distilled water when heated and hand-shaken. Eventually, a gelatinous matrix is yielded that appears to be crystalline when viewed under a polarizing microscope. In the presence of 0.1 *N* HCl or pH 7.0 phosphate buffer, these lipid formulations do not appear to swell in the presence of distilled water when heated and hand-shaken, but remain as large oil/solid particles in these solutions. In contrast, these lipid formulations in the presence of distilled water and aqueous bile salts, with heat and hand-shaking, yield micron sized particles when viewed under a polarizing microscope. A conclusion that can be drawn from these observations is that the ionic species in the aqueous medium affect the size and constitution of particles formed from these lipid formulations. In particular, the anion types can significantly alter lipid particle formation, constitution and size.

It is known that, in addition to bile salts, the principal anion in the upper region of the small intestine is bicarbonate. It has been found that when this anion is present in sufficient quantities in the aqueous medium of the lipid formulations, submicron particles can be formed. The bicarbonate is incorporated in the compositions of the present invention by directly mixing the bicarbonate with the lipid components, or, preferably, by dissolving salts of this anion, such as sodium bicarbonate, potassium bicarbonate, etc., in the aqueous environment to which the previously mixed lipid components of the compositions have been placed. When the mixed lipid colloidal particles are formed by the shearing operation, bicarbonate is integrally included in the particulate form of the compositions.

Other agents which effectively combine with the mixed lipid compositions to form submicron sized colloidal particles may be termed "titrating agents". These include other salts of bicarbonate, sodium hydroxide, potassium hydroxide, and the like. When titrating agents are used which are strongly basic, the pH of the aqueous environment must be closely monitored to avoid base hydrolysis of the lysophosphatidylcholine and monoglyceride components of the lipid composition. Titrating agents which include divalent cations such as calcium or magnesium may also be used in preparation of submicron particles of the mixed lipid composition.

If bile salts are additionally present in sufficient quantities in the aqueous environment, the already submicron particles can be even further reduced in size. Examples of bile salts that will reduce the size of the mixed lipid-titrating agent colloidal particles are sodium taurocholate and sodium glycocholate. The bile salts can be added to the non-aqueous mixed lipid-titrating agent mixture, or, preferably, are added to the aqueous environment in which the lipid components and bicarbonate have been combined. The bile salts then become incorporated in the colloidal particles of the compositions when these particles are formed by the shearing operation. Other biologically compatible surfactants may be substituted for the bile salts in these compositions. Mixtures of more than one biologically compatible surfactant, including bile salts, may also be used.

The compositions of this invention are preliminarily made according to the following method. The component lipids are weighed and mixed, with or without heat, to attain liquid homogeneity. When a drug is incorporated, it is added and dissolved, with or without heat, in the lipid mixture. A uniform state is indicated by the absence of any solids at the appropriate temperature for the mixture to be a liquid and by the absence of any schleiren. A schleiric effect will be more apparent at greater concentrations of the drug in the lipid mixture if a drug is included. The formulation is stable to several freeze-thaw cycles; the appearance of solids or schleirin may indicate instability of the formulation.

A second preliminary method of making the formulation involves dissolving the component lipids and drug, if a drug is incorporated, in a solvent or mixture of solvents and mixing to attain homogeneity. The solvents are removed, in vacuo or by other suitable methods. The criteria for a suitable formulation are the same as noted above.

A desired amount of an above preliminary formulation is placed in an aqueous environment. This aqueous environment is predominately water. Other substances can be present without altering the basic compositions. Examples of these other substances are pH buffering materials, amino acids, proteins, such as albumin or casein, and viscosity enhancers such as xanthine gums or gum arabic. The only criterion for the presence of these other substances is that they not substantially interfere with or alter the forces which cause the individual components of the composition to form the colloidal particles of the composition. A third preliminary method of making the formulation involves combining the component lipids and drug, if it is incorporated, in an aqueous environment without prior mixing of the component lipids.

Bicarbonate, or other titrating agent, is added to the aqueous environment by dissolving a desired amount of bicarbonate salt, or other titrating agent, in the aqueous environment either before or after the preliminary formulation has been placed there.

The component lipid mixture in the aqueous environment is then subjected to shearing forces by an appropriate means. Typically, these shearing forces are achieved with a sonicator or a microfluidizer. The shearing operation is performed at an appropriate energy and for a time sufficient to yield homogeneous lipid-containing particles of the desired size. As noted in a below exemplification, the amount of bicarbonate relative to the amount of lipid formulation is important in determining the ultimate size of the mixed lipid-bicarbonate colloidal particles. Below a molar ratio of 1.4:1 (bicarbonate:mixed lipid formulation) the mixed lipid bicarbonate colloidal particle size will be larger than approximately 120 nm. Between a molar ratio of 1.4:1 and 7:1, the mixed lipid-bicarbonate colloidal particle size will be between approximately 120 nm and approximately 70 nm, depending on the molar ratio of bicarbonate to mixed lipid formulation. The bicarbonate can be added gradually or all at one time as the shearing procedure is performed. Alternatively, the bicarbonate can be added before the shearing procedure is performed.

To obtain smaller submicron particles, bile salts, or other biologically compatible surfactants, at an appropriate molar ratio (bile salt:mixed lipid formulation) can be added to the aqueous medium before, concurrently, or after the bicarbonate is added. The molar ratios of bile salt to mixed lipid formulation as well as bicarbonate to mixed lipid formulation can be any independent value, provided each of them is at least about 1:1 (i.e., the bile salt concentration or the bicarbonate concentration should be at least the same as the mixed lipid concentration). That is, the bile salt:mixed lipid formulation molar ratio as well as the bicarbonate:mixed lipid formulation molar ratio can be independently changed, resulting in an accompanying change in the mixed lipid-bicarbonate-bile salt colloidal particle size. However, to achieve mixed lipid-bicarbonate-bile salt colloidal particles of 10 nm or less, the molar ratio of bile salt or other biologically compatible surfactant to mixed lipid formulation should be at least about 10:1 and the molar ratio of titrating agent to mixed lipid formulation should be at least 7:1. Again, the bile salts can be added gradually or all at one time before or while the shearing operation is performed.

Mixtures of mixed lipid-bicarbonate colloidal particles, and optionally, mixed lipid-bicarbonate-bile salt colloidal particles, can be made from the individual colloidal particle formulations. These mixtures have different colloidal particle sizes which can affect their emptying time from the stomach and which have different fat processing and uptake rates from the small intestine to the bloodstream. These colloidal particle mixtures, thus, have prolonged uptake attributes.

As disclosed herein, colloidal particles of different sizes can be prepared. When it is desired to provide sustained delivery of a bioactive agent, a mixture of at least two separately prepared sets of particles having different size distributions is prepared. The size distributions of the sets of mixed lipid colloidal particles can be predetermined by choosing the molar ratios of titrating agent and/or biologically compatible surfactant added to the mixed lipid compositions, and are a distribution of particle sizes spaced around an average particle size. The particle size distributions can be envisioned as having a generally bell-shaped curve. When two or more sets of particles are combined, the curves which represent the particle size distributions of those sets can overlap, i.e. some of the particles in a first set of particles may have the same size as some of the particles in a second set of particles. To provide sustained delivery, preferably at least 80% of the particle size distribution of the first set of particles is less than the average particle size of the set set of particles, and at least 80% of the particle size distribution of the second set of particles is greater than the average particle size of the first set of particles. Thus sustained delivery is achieved by the differential rate of processing and uptake in the stomach and intestine of the different size mixed lipid particles.

The mixed lipid colloidal particles are useful for delivery of a bioactive agent. As used herein, "bioactive agent" includes any molecule or compound which affects or is required for metabolic function. For example, bioactive agents include therapeutic compounds (e.g. drugs), foods such as saturated or polyunsaturated fatty acids or fats (e.g. as a source of calories), and compound which are substrates, catalysts or cofactors for metabolic processes. Bioactive agents can be added during the preparation of the colloidal particles, e.g., before shearing force is applied. Although not traditionally regarded as bioactive agents, the mixed lipid particles can be used to deliver diagnostic agents such as fluorescent compounds and/or radioactive compounds.

Virtually any bioactive agent can be used according to the invention, as will be recognized by one of ordinary skill in the art. Important therapeutic categories which would most benefit from this drug delivery system include anti-infectives, cardiovascular (hypotensives and vasodilators), analgesics, psychotherapeutics, anti-convulsives, diuretics, hormones, anti-neoplastics and vitamins. Other therapeutic categories which would benefit from this drug delivery system include: adrenergic agent; adrenocortical steroid; adrenocortical suppressant; alcohol deterrent; aldosterone antagonist; anabolic; analeptic; analgesic; androgen; anorectic; antagonist; anterior pituitary suppressant; anthelmintic; anti-acne agent; anti-adrenergic; anti-allergic; anti-amebic; anti-androgen; anti-anemic; anti-anginal; anti-anxiety; anti-arthritic; antiasthmatic; anti-atherosclerotic; antibacterial; anticholelithic; anticholelithogenic; anticholinergic; anticoagulant; anticoccidal; anticonvulsant; antidepressant; antidiabetic; antidiarrheal; antidiuretic; antidote; anti-emetic; anti-epileptic; anti-estrogen; antifibrinolytic; antifungal; antihemophilic; antihemorrhagic; antihistamine; antihyperlipidemia; antihypertensive; antihypotensive; anti-infective; anti-infective, topical; anti-inflammatory; antikeratinizing agent; antimalarial; antimicrobial; antimigraine; antimitotic; antimycotic, antinauseant, antineoplastic, antineutropenic, antiobessional agent; antiparasitic; antiparkinsonian; antiperistaltic, antipneumocystic; antiproliferative; antiprostatic hypertrophy; antiprotozoal; antipruritic; antipsychotic; antirheumatic; antischistosomal; antiseborrheic; antisecretory; antispasmodic; antithrombotic; antitussive; anti-ulcerative; anti-urolithic; antiviral; appetite suppressant; benign prostatic hyperplasia therapy agent; blood glucose regulator; bone resorption inhibitor; bronchodilator; carbonic anhydrase inhibitor; cardiac depressant; cardioprotectant; cardiotonic; cardiovascular agent; choleretic; cholinergic; cholinergic agonist; cholinesterase deactivator; coccidiostat; cognition adjuvant; cognition enhancer; depressant; diagnostic aid; diuretic; dopaminergic agent; ectoparasiticide; emetic; enzyme inhibitor; estrogen; fibrinolytic; fluorescent agent; free oxygen radical scavenger; gastrointestinal motility effector; glucocorticoid; gonad-stimulating principle; hair growth stimulant; hemostatic; histamine H2 receptor antagonists; hormone; hypocholesterolemic; hypoglycemic; hypolipidemic; hypotensive; imaging agent; immunizing agent; immunomodulator; immunoregulator; immunostimulant; immunosuppressant; impotence therapy adjunct; inhibitor; keratolytic; LHRH agonist; liver disorder treatment; luteolysin; memory adjuvant; mental performance enhancer; mood regulator; mucolytic; mucosal protective agent; mydriatic; nasal decongestant; neuromuscular blocking agent; neuroprotective; NMDA antagonist; non-hormonal sterol derivative; oxytocic; plasminogen activator; platelet activating factor antagonist; platelet aggregation inhibitor; post-stroke and post-head trauma treatment; potentiator; progestin; prostaglandin; prostate growth inhibitor; prothyrotropin; psychotropic; pulmonary surface; radioactive agent; regulator; relaxant; repartitioning agent; scabicide; sclerosing agent; sedative; sedative-hypnotic; selective adenosine Al antagonist; serotonin antagonist; serotonin inhibitor; serotonin receptor antagonist; steroid; stimulant; suppressant; symptomatic multiple sclerosis; synergist; thyroid hormone; thyroid inhibitor; thyromimetic; tranquilizer; amyotrophic lateral sclerosis agent; cerebral ischemia agent; Paget's disease agent; unstable angina agent; uricosuric; vasoconstrictor; vasodilator; vulnerary; and wound healing agent.

Important examples for this drug delivery system are:
Anti-infectives: cephalosporins, chloramphenicols, erythromycins, penicillins, tetracyclines and aminoglycosides.
Cardiovasculars: propranolol, digitalis, quinidine, methyl DOPA, prazosin, nifedipine, nitroglycerin, isosorbide dinitrate, nadolol, diltiazem and verapamil.
Analgesics: aspirin, naproxen, ibuprofen, indomethacin, codeine, and meperidine.
Psychotherapeutics: tricyclic compounds such as doxepin, phenothiazine. compounds such as chlorpromazine, and benzodiazepin compounds such as diazepin.
Anti-convulsants: phenytoin, carbamazepine, and ethosuximide.
Diuretics: chlorothiazide, furosemide, metolazone, spironolactone, and triamterene.
Hormones: corticosteroids, androgens, contraceptive, estrogens, progesterones, and antidiabetic agents.
Vitamins: vitamin A, vitamin D, vitamin E, and vitamin K.
Anti-neoplastics: azathiopine, procarbazine, megestrol acetate, melphalan, cyclophosphamide, BCNU, CCNU, hydroxyurea, uracil mustard, and doxorubicin.

In addition, delivery of lipophilic analogs of these drugs is greatly benefited by this delivery system.

Biologically compatible surfactants can be added at any time to the aqueous medium containing the lipid formulation and bicarbonate (optionally also containing the bile salt). Examples of biologically compatible surfactants include polysorbates such as TWEEN 20, TWEEN 40, TWEEN 60, and TWEEN 80, polyethylene glycols (PEG), sorbitan esters, dialkyl sodium sulfosuccinates, and the like. Other examples include sorbitan monolaurate, polyoxyethylene-4-lauryl ether, polyethylene glycol 400 monostearate, polyoxyethylene-4-sorbitan monolaurate, polyoxythylene-20-sorbitan monooleate, polyoxyethylene-20-sorbitan monopalmitate, polyoxyethylene-20-sorbitan monolaurate, and polyoxyethylene-40-stearate. These surfactants can be added before or after the shearing operation, along with or in place of like salts to obtain desired colloidal particle size.

The mixed lipid-titrating agent or the mixed lipid-titrating agent-bile salt colloidal particles are stable and can be stored under normal storage conditions. When a bioactive agent, e.g. a drug is incorporated in either of these compositions, the colloidal particles serve as a vehicle for transporting the drug to the intestinal mucosal cells following oral administration of the drug-containing particles to an individual. Sustained drug release compositions can be made by using mixtures of different size colloidal particles, as previously described, with the drug incorporated within the different size colloidal particles. Differential processing and uptake rates for the different size particles provides a more sustained uptake rate for the incorporated drug. These drug-containing colloidal particles can be packaged, for example, in individual containers for oral administration of specific dosages of the incorporated drug. An individual simply opens the packaging container and swallows its contents to achieve the oral administration of the drug-containing colloidal particles.

Likewise, the mixed lipid-bicarbonate or the mixed lipid-bicarbonate-bile salt compositions can serve as a source of calories, polyunsaturated fat and choline when administered without an incorporated drug. Again, an individual simply swallows the contents of a container, or eats a candy/wafer bar or other food product, that has a specific amount of the mixed lipid formulation to achieve oral administration of the desired composition.

The mixed lipid-titrating agent or the mixed lipid-titrating agent-surfactant compositions, with or without a constituent drug, also can be topically applied to the skin of an individual or applied by inhalation. Such application provides a source of lipids, and drug if included, to the skin surface and/or lung mucosal surface for whatever purpose is desired.

When administered, the compositions of the present invention can be administered in pharmaceutically acceptable preparations. Such preparations may routinely contain pharmaceutically acceptable concentrations of salt, buffering agents, preservatives, compatible carriers, and the like.

As used herein, an effective amount of bioactive agent administered via the mixed lipid colloidal particles of the invention is a dosage large enough to produce the desired therapeutic effect. An effective amount is not, however, a dosage so large as to cause adverse side effects. Generally, an effective amount of a bioactive agent may vary with the subject's age, condition, weight and sex, as well as the extent of the condition being treated, and can be determined by one of skill in the art. The dosage may be adjusted by the individual practitioner in the event of any complication.

The present invention is illustrated by the following examples which are not intended to be limiting of the invention.

### COMPARATIVE EXAMPLE I: Formation of Submicron Sized Particles of Lipid Formations:

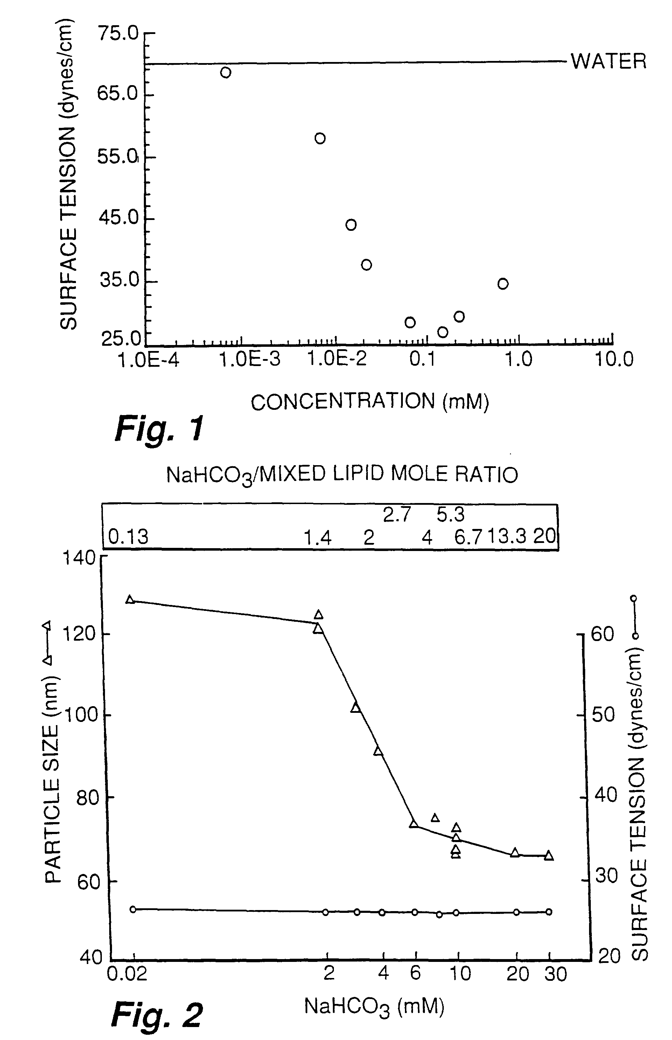

The following lipids were mixed together to yield a non-aqueous lipid mixture: soy lysophosphatidylcholine (LPC), 18:1 monoolein monoglyceride (MG), and 18:1 oleic acid fatty acid (FA). The sources of these lipids were: Avanti Polar Lipids, 5001A Whitling Drive, Pelham, AL 35124 for LPC, and Nu-Chek-Prep, Inc., P.O. Box 295, Elysian, MN 56028 for MG and FA. The molar ratio of these lipid components was 1:3:3 for LPC:MG:FA. This non-aqueous lipid mixture was put into water at LPC concentrations ranging from 10⁻³ to 1 mM. Since the molar ratio of LPC:MG:FA was 1:3:3, the total lipid mixture molar concentrations also ranged from 10⁻³ to 1 mM in the water environment. These formulations were then subjected to probe sonication (Cole-Parmer, 4710 Series with a S&M 10 86 tip, 1.25 minutes at full power output). The surface tension (dynes/cm) of these mixed lipid formulations was measured by determining the time between drops. Using this technique, the critical micelle concentration of this mixed lipid formulation was found to be about 0.1 mM (See Figure 1).

Particle sizes were measured of a 1.5 mM concentration of LPC (and also total lipid mixture) of the 1:3:3 LPC:MG:FA formulation in water after probe sonication was performed. The particle sizes were measured with either a Nicomp Analyzer or a Brookhaven Particle Sizer. After the initial probe sonication, the particle size was approximately 170 nm. Sodium bicarbonate, NaHCO₃ was incrementally added, sonication was continued and particle size was monitored. As the molar ratio of bicarbonate to lipid formulation (bicarbonate:lipid) approached 1.4:1, the particle size approached approximately 120 nm. When the bicarbonate:lipid molar ratio was increased to 7:1, the particle size decreased to approximately 70 nm. Between these bicarbonate:lipid molar ratios, intermediate size particles of the lipid formulation were observed (see Figure 2). As the bicarbonate:lipid molar ratio was further increased, the particle size did not significantly change.

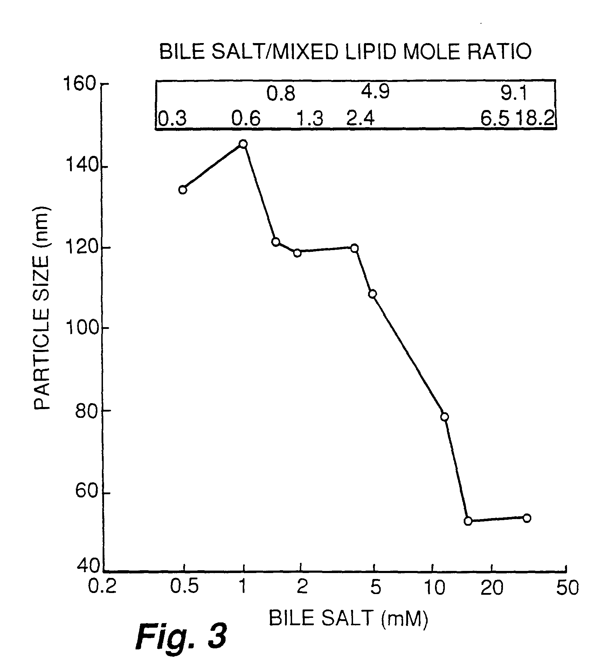

In another experiment, soy lysophosphatidylcholine (LPC), 18:1 monoolein monoglyceride (MG), and 18:1 oleic acid fatty (FA) acid from the same sources as in the first experiment were mixed together to yield a non-aqueous lipid mixture with a molar ratio of 1:3:3 LPC:MG:FA. The non-aqueous lipid mixture was put into water so the LPC (and also total lipid mixture) molar concentration was about 1.7 mM. This formulation was then subjected to either probe sonication (Cole-Parmer sonicator) or shearing by action of a Microfluidizer (Model 110T, 2 passes at 70 psi). After the shearing operation, the particle size was approximately 150 nm for the 1:3:3 formulation. The bile salt, sodium taurocholate, was gradually added and shearing was continued. The particle size was monitored as the bile salt was added. The particle size was reduced to approximately 100 nm while the bile salts were in their monomeric state (i.e., less than about 5 mM) and the molar ratio of bile salt to mixed lipid was about 5:1. As more bile salt was added, the particle size for this formulation decreased to approximately 50 nm when the bile salts were primarily in their micellar state (i.e., greater than about 5 mM) and the molar ratio of bile salt to mixed lipid was about 9:1. Between these bile salt:mixed lipid molar ratios, intermediate size particles of the lipid formulations were observed (see Figure 3).

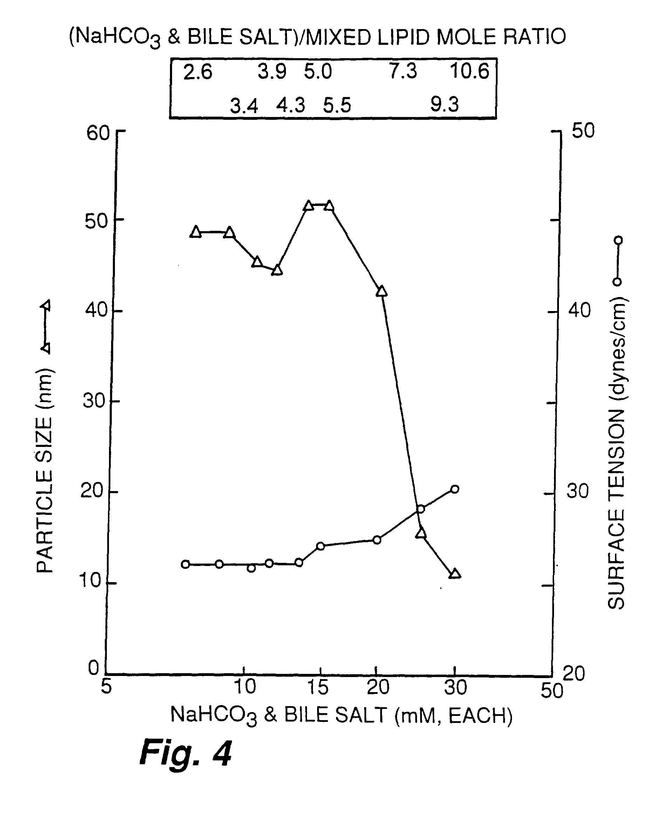

When bicarbonate was added with sonication to the formulations of the latter experiment, the particle size was further reduced to approximately 10 nm or less as the bicarbonate:lipid molar ratio was increased to at least 7:1. When the bile salt, sodium taurocholate, was added with sonication to the formulation of the second experiment, the particle size was further reduced to approximately 10 nm or less as the bile salt:lipid molar ratio was increased to 10:1, i.e., as the bile salts reached their critical micellar concentration (achieving the micellar state). That is, when both the bicarbonate ion and bile salt reached their optimal concentrations for forming the smallest size particles of the lipid formulations, the particle size was approximately 10 nm or less (see Figure 4, where the concentration of LPC, and also total lipid mixture, was about 2.7 mM for the 1:3:3 LPC:MG:FA formulation).

### COMPARATIVE EXAMPLE 2: Incorporation of Drugs in the Colloidal Particles:

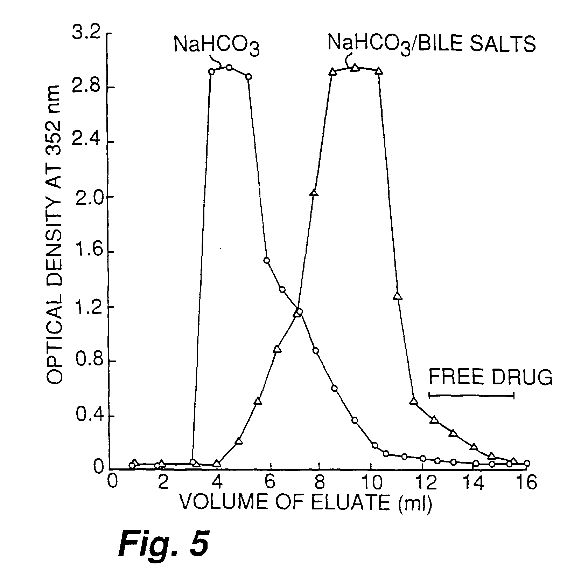

Fenretinamide was formulated with the mixed lipid LPC:MG:FA (1:3:3 molar ratio) using the solvent method of preparation. The molar concentration of fenretinamide was 0.8 with respect to LPC (and also total lipid mixture) in the mixed lipid(drug)-formulation. This non-aqueous mixed lipid(drug) formulation was put into an aqueous environment so the LPC (and also total lipid mixture) concentration was about 1.3 mM. The aqueous environment contained either bicarbonate at a concentration of 12.5 mM (i.e. a molar ratio of about 10:1 for bicarbonate:(LPC in mixed lipid) or bicarbonate and bile salt at respective concentrations of 12.5 mM (i.e. molar ratios of 1:10:10 for LPC in mixed lipid:bicarbonate:bile salt). Colloidal particles of this mixed lipid (fenretinamide) with bicarbonate or with bicarbonate and bile salt were made by the method described in Example 1. This drug is hydrophobic in nature and tends to reside in the hydrocarbon region of the mixed lipid-bicarbonate or mixed lipid-bicarbonate-bile salt formulations. Upon size exclusion chromatography on a Sepharose 4B column, the drug remained associated with the mixed lipid-bicarbonate or with the mixed lipid-bicarbonate-bile salt particles (see Figure 5). Free drug, i.e., without the presence of the particles, eluted from the column with a distinct elution profile in the region identified as 'free drug' in Figure 5. The smaller size of the mixed lipid(drug)-bicarbonate-bile salt particles compared with the mixed lipid(drug)-bicarbonate particles is noted from the longer retention before elution from the size exclusion column.

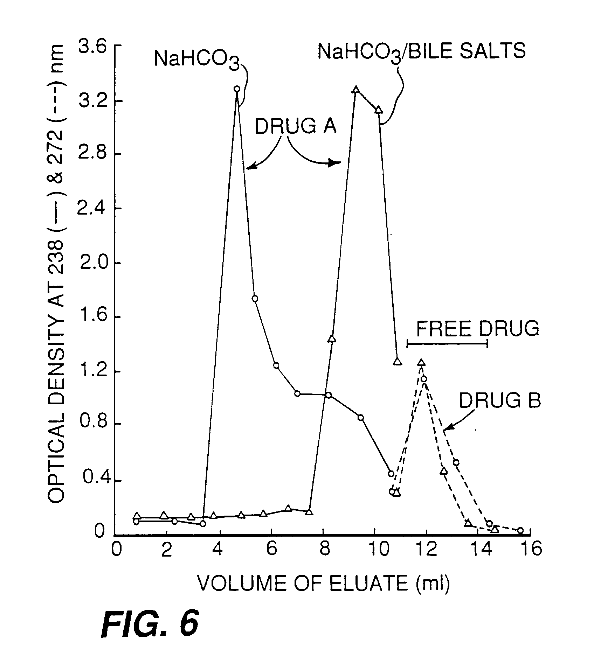

Diltiazem, a benzothiazepine, was formulated with the mixed lipid LPC:MG:FA (1:3:3 molar ratio) using the solvent method of preparation. Colloidal particles of this mixed lipid (diltiazem) formulation with either bicarbonate or bicarbonate and bile salt were made by the method described in the preceding experiment. This drug is hydrophobic in nature and tends to reside in the hydrocarbon region of the mixed lipid-bicarbonate formulations. Upon size exclusion chromatography on a Sepharose 4B column, the drug remained associated with the mixed lipid-bicarbonate particles as well as with the mixed lipid-bicarbonate-bile salt particles. Free drug, i.e. without the presence of the particles, eluted from the column with a distinct elution profile when compared with the drug associated with the mixed lipid-bicarbonate particles. (See Drug A and 'Free Drug' of Figure 6).

In a separate experiment, hydrochlorothiazide (HCTZ) was formulated with the mixed lipid LPC:MG:FA (1:3:3 molar ratio) using the solvent method of preparation. This drug is not soluble *per se* with just monoglycerides and fatty acids. However, colloidal particles of this mixed lipid(HCTZ) formulation with bicarbonate were made by the method described in the first experiment of this Example. Size exclusion chromatography of these mixed lipid(HCTZ) bicarbonate particles on a Sepharose 4B column showed the elution profile of HCTZ as free HCTZ. This indicates that HCTZ probably resides in the polar regions of the mixed lipid-bicarbonate formulations and becomes free drug when bicarbonate is present. Next, the mixed lipid(HCTZ) formulation and mixed lipid(diltiazem) formulation of the preceding experiment were mixed together at about a 1:5 weight ratio of the respective formulations. This 'super mixture' was then sonicated in either an aqueous bicarbonate solution or an aqueous bicarbonatebile salt solution and the resulting materials were eluted by size exclusion chromatography from a Sepharose 4B column. Mixed lipid (diltiazem)-bicarbonate colloidal particles or mixed lipid (diltiazem)-bicarbonate-bile salt colloidal particles (Drug A in Figure 6) and free HCTZ (Drug B in Figure 6) were eluted. These results show that with this technique of making mixed lipid(drug)-bicarbonate colloidal particles or mixed lipid(drug)-bicarbonate-bile salt colloidal particles, together with size exclusion chromatography, one can approximate the stability of mixed lipid-drug formulations to the milieu of the GI tract.

### EXAMPLE 3: Comparison of Fat Uptake by Humans When the Fat is in Random. Eutectic Matrix and Colloidal Particle (Colloidal Eutectic Matrix) Formulations:

### Formulation Material:

The substrates used in the colloidal eutectic matrix, eutectic matrix and triglyceride-control formulations were food-grade, commercially available components, except for soy lysophosphatidyl choline and the C13-labeled fats. The fatty acids (EMERSOL 6313 oleic and EMERSON 6332 stearic) were good grade (chick edema factor free) and were obtained from Henkel Corporation Emery Group, monoglycerides (Dimodan OK and Dimodan LSK) from Grindsted Products, Inc., maltodextrin carbohydrate (Maltrin 500) from Grain Processing Corporation, proteins (whey hydrolysate WPH 916 and calcium casemate ALANATE 310) from New Zealand Milk Products, Inc. and the triglycerides fats (corn, safflower and lard) from local food stores. Vitamin and mineral mixes were formulated by Fortitech, Inc.

Soy lysophosphatidyl choline, a Type 4 excipient, was purchased from Avanti Polar Lipids, Inc., which is prepared under their FDA Drug Master File #5916, dated November 1987. The ¹³C-labeled lipids: oleic acid (1-C13, 99%) and triolein (1,1,1-C13, 99%) were purchased from Cambridge Isotope Laboratories.

### Eutectic Matrix, Colloidal Particle (Colloidal Eutectic Matrix) and Triglyceride-Control Formulations:

The colloidal eutectic matrix formulation is composed of a eutectic matrix of structured lipids, with protein, carbohydrates, vitamins and minerals. The eutectic structured lipid is a stable matrix and has an approximate molar ratio of lysophosphatidylcholine, monoglyceride and fatty acid of 1:3:3. During the preparation of the eutectic matrix fat, C13-oleic acid was first added to the fatty acid and mixed to uniformity. Sequentially, monoglycerides and soy lysophosphatidyl choline were added and heated yield a uniform C13-labeled eutectic matrix fat. This fat was incorporated into the colloidal eutectic matrix formulation by two different methods. In the control population, the C13-labeled eutectic matrix fat was initially emulsified in an aqueous NaHCO₃ solution and further emulsified in an aqueous suspension of the proteins, then frozen and lyophilized to yield a powdered mixture. The carbohydrate, vitamins and minerals were premixed in the powdered, dry state. These two powdered premixes were stored at -20°C until used. At that time, defined amounts of both premixes were blended with sufficient ice and water to yield a "milk shake" for oral administration. The control group reported that this aqueous colloidal eutectic matrix formulation had a bitter taste and might be objectionable and be rejected by the CF patients. Therefore, a second formulation approach was used for the CF patients. The eutectic lipid matrix does not have a bitter taste per se, but does yield an objectionable bitter taste in the presence of aqueous carbohydrates. Therefore, the colloidal eutectic lipid matrix (the eutectic matrix emulsified with NaHCO₃) was initially mixed with an equal amount of water and with a powdered calcium chloride and calcium caseinate premix. The resultant colloidal eutectic matrix paste was then blended in water/ice with the powdered whey protein, maltrin, vitamin and mineral premix and consumed as a "milk shake". The physical size of the structured lipids would be larger in the eutectic matrix "milk shake" than in the emulsified "milk shake".

The triglyceride-control of the colloidal eutectic matrix and eutectic matrix formulations were prepared similarly to that for the latter formulations, except that C13-labeled triolein was used instead of C13-labeled oleic acid.

The colloidal eutectic matrix, eutectic matrix and triglyceride-control formulations were also prepared without C13-labeled substrates in order to determine the natural abundance of ¹³CO₂ being derived from the colloidal eutectic matrix, eutectic matrix and triglyceride-control formulations.

The caloric distribution of the colloidal eutectic matrix and triglyceride-control formulations were equivalent; i.e. 41% from fat, 41% carbohydrates and 18% protein. The profile of the major fatty acids were nearly equivalent, containing approximately 0.5 mmol/g of C18:2n6 (linoleic), 0.6 mmol/g C18:ln9 (oleic), 0.2 mmol/g C18:0 (stearic) and 0.4 mmol/g of C16:0 (palmitic). The mole ratio of the unsaturated to saturated fatty acids was 1.95 and 2.09 for the colloidal eutectic matrix and the triglyceride-control formulations respectively. The concentration of C13-labeled oleic acid and triolein was 13 mg/g of formulation. The formulations were dosed at 2g/kg of body weight, the C13-labeled substrates at 26mg/kg and the fatty acids at 2 mmol/kg.

### Analytical Procedures:

Mass spectrometric analysis of ¹³CO₂ in breath samples and the calculation of expired ¹³CO₂ as percent dose per hour and area under curve (AUC) (% dose-hours) were described by Watkins et al. (Gastroent, 82: 911-917 (1982)). The analyses for ¹³CO₂ were performed by Cambridge Isotope Laboratories.

Gas liquid chromatographic analysis of the acyl fatty acids in the lipid components of the colloidal eutectic matrix and triglyceride formulations used the procedure of G. Lepage et al. (J. Lipid Res. 30: 1483-1490(1989)).

### Subjects and Study Protocol:

Fifteen cystic fibrotic (CF) patients, in stable health (excluded cirrhosis, diabetes, meconium ileus and liver disease), age 10-19 years, either sex and previous acknowledged compliance were stratified according to the severity of their steatorrhea (less than 10 pancrease pills/day and greater than 10 pancrease/day). Pancrease was not consumed during the overnight fast and the test period. In addition, five individuals with normal fat absorption were selected using similar criteria as for the CF subjects. The breath collections were conducted over an eight hour period for all control individuals and seven CF subjects, receiving the eutectic matrix formulation, over a twelve hour period. The two C13-labeled formulations were administered in a random sequence to each individual with a two weeks interval between dosing for CF patients and 4 days for normal subjects. The study was approved by two Human Studies Committees and written consent was obtained from all individuals and parents.

All the control subjects were compliant to the protocol for both the colloidal eutectic matrix, eutectic matrix and triglyceride-control formulations. In the cystic fibrosis group consuming the triglyceride formulation, subject 16 withdrew from the study, subject 19 consumed his normal pancrease supplements and subject 18 was an outlier (greater than 2 SEM) for undetermined reasons. In the CF/eutectic matrix group, subject 7 withdrew from the study, subject 8 vomited during the consumption of the formulation and subject 20 was given a spoiled eutectic matrix preparation. The other CF subjects were all compliant for both formulations and there was a general consensus that they did not like either "milk shake".

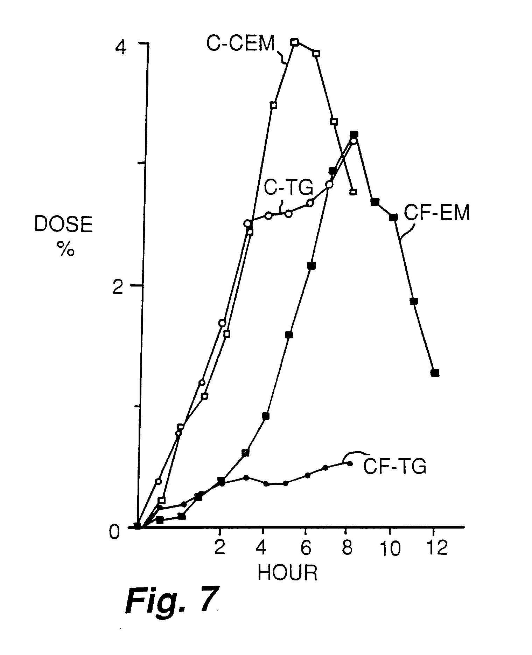

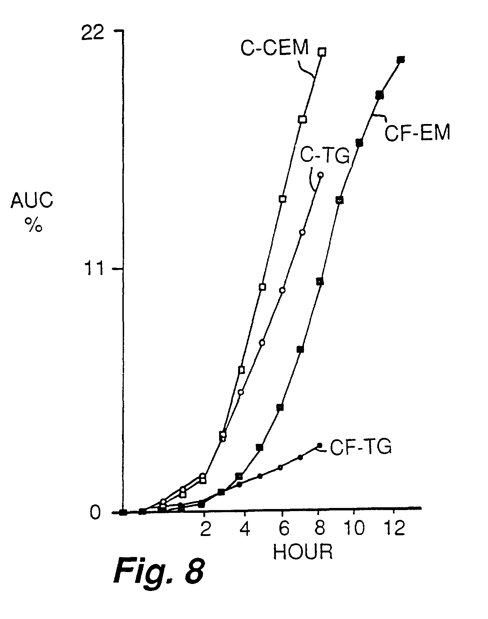

The absorption of 13C-labeled fat formulations in control and cystic fibrosis individuals are shown in Figures 7 and 8. Briefly, the colloidal eutectic matrix (CEM) and the eutectic matrix (EM) fat shows a single absorption peak in both the control and CF patients but delayed approximately 3 hours in CF patients (Figure 7). Even with this delay, the absorption area under the curve (AUC) for colloidal eutectic matrix and the eutectic matrix fat were equivalent for the two groups. The absorption of the triglyceride (TG)-control fat in control and CF patients (Figures 7 and 8) was comparable to that described by Watkins et al. for normal and pancreatic insufficient individuals. It is readily apparent that the CF patients absorbed the eutectic matrix fat organized structured lipid in contrast to the minimal digestion and absorption of the triglyceride-control fat. A statistical analysis indicated that the decreased absorption of triglyceride in CF patients relative to both TG in controls and EM in CF patients was significantly different (p <0.001). In contrast, there was no significant difference in the absorption of the colloidal eutectic matrix or eutectic matrix between control and CF patients and of triglyceride in control subjects.

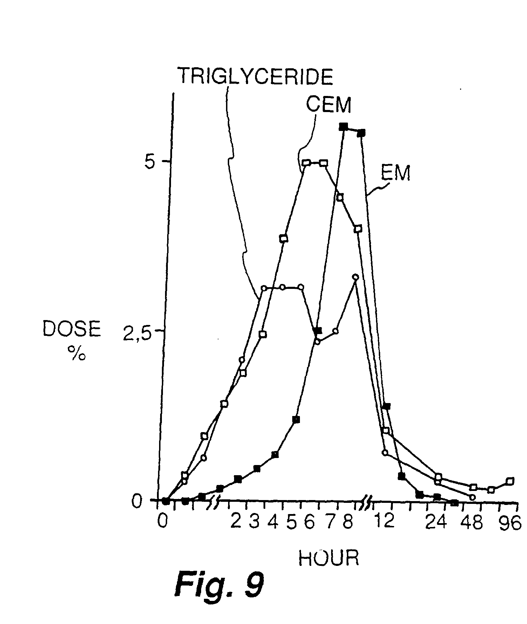

The delayed absorption of the eutectic matrix lipids in the CF patients compared to control subjects could arise from delayed gastric emptying in CF or from differences in the physical state of the eutectic matrix lipids; i.e. emulsified colloidal eutectic matrix or eutectic matrix in controls and eutectic matrix in CF (see Methods). Control subject #1 had previously provided breath analyses over 48/96 hours for both the CEM and TG formulations. She agreed to evaluate the lipids as a eutectic matrix formulation over an extended period. These data are shown in Figure 9. It is readily apparent that the physical state of the eutectic matrix lipids within the formulation is an important factor in the time for maximum absorption but is not of any consequence in the total absorption of the readily absorbable lipid matrix.

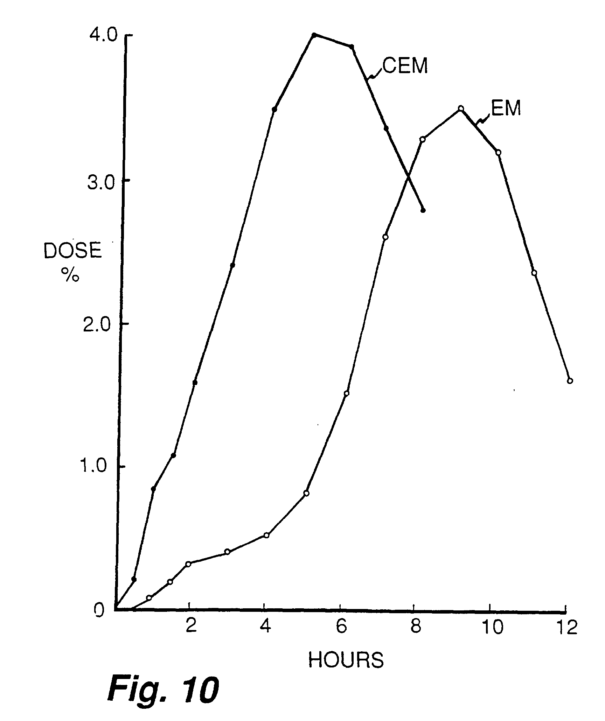

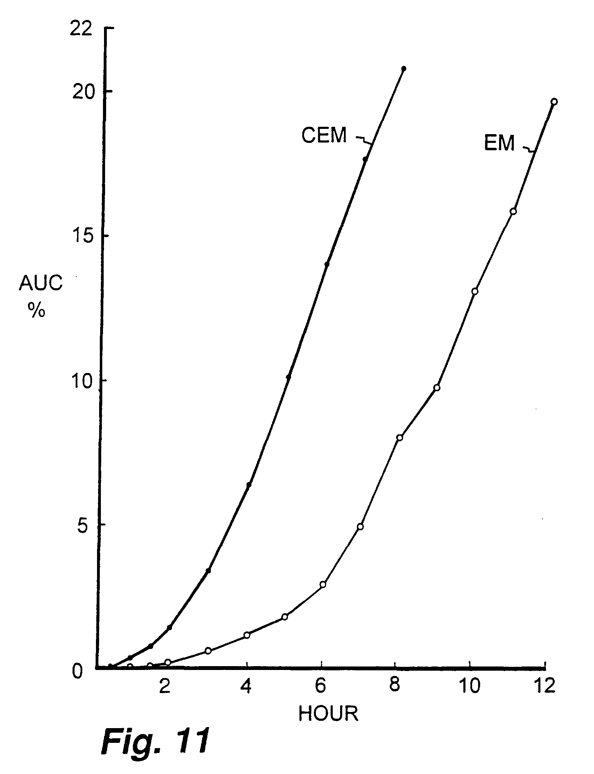

In a further study, the absorption of the eutectic matrix lipids as either eutectic matrix (EM) or colloidal eutectic matrix (CEM) fat in five control individuals was determined. The results of this study are graphically shown in Figures 10 and 11. It can readily be seen that smaller particle sizes (CEM) are absorbed more rapidly than larger particle sizes (EM). These results further demonstrate that the physical state of the eutectic matrix lipids affects the time of maximum absorption of the fats but does not seriously affect the total absorption of the absorbable lipid matrix.

In comparing the absorption AUC for both TG and CEM formulations in individual control subjects, it was apparent that the absorptions were comparable (R = 1.0). In the CF patients, when there was minimal absorption (<1% AUC) of triglyceride lipids the absorption of eutectic matrix lipids was lower (ca. 14-15% AUC) and when there was some absorption (2-5% AUC) of the triglyceride lipids the absorption of the eutectic matrix lipids was greater (>20% AUC). A correlation was established between TG and eutectic matrix absorption in CF at r = 0.83 and p<0.02. This indicates that the readily absorbable lipid characteristic is an important factor in the design of this new lipid formulation.

In this study, it was shown that cystic fibrosis patients poorly absorbed ingested triglycerides in comparison to normal subjects. The AUC (% dose-6 hours) and the peak dose % were comparable to those previously reported by Watkins et al. The colloidal eutectic matrix or eutectic matrix structured lipids were well absorbed in both normal and cystic fibrosis individuals, showing an absorptive maximum at 5-6 hours for controls and 8 hours for CF. This difference was shown to be related to the difference in physical size of the eutectic matrix lipid particles; i.e. the smaller "emulsified" lipid formulation (CEM) was absorbed earlier than the larger "paste" lipid formulation (EM). This delay in absorption of the larger "paste" lipid formulation was also observed in a control subject. The absorption of triglycerides in control subjects showed two absorptive peaks at 3-4 and 6-8 hours. The absorptive AUC for the triglyceride in control subjects was somewhat (ca. 5%) reduced compared to the colloidal eutectic matrix in control subjects. Statistically, the absorption of colloidal eutectic matrix or eutectic matrix structured lipids in both control and CF subjects and of triglyceride lipids in control subjects were comparable whereas the absorption of triglyceride lipids in cystic fibrosis was statistically (p<.001) decreased compared to absorption of eutectic matrix structured lipids in CF patients. The capacity to absorb triglyceride and eutectic matrix fat in CF patients was positively correlated (r = 0.82, p <0.02). The absorption of EM structured lipids in CF patients was not affected by the severity of the steatorrhea, since there was no difference between the low (<10) or high (>10) pancrease taken by the patient. The sex of the CF patient was also not a factor.

It is concluded that the colloidal eutectic matrix or eutectic matrix structured lipids are useful for the delivery of readily absorbable fat calories and for the prevention and treatment of essential fatty acid and fat soluble vitamin deficiency in cystic fibrosis patients as well as in other disease conditions with malabsorption or malnutrition. The colloidal eutectic matrix or eutectic matrix structured lipids are also useful for the delivery of chlorine to the elderly, pregnant and lactating women, newborns, patients on total parenteral nutrition, and subjects having neurodegenerative disease, liver damage, or following strenuous exercise.

The clinical implications of this preliminary study are considerable. malnutrition in CF patients has been associated with decreased ventilatory drive, impaired pulmonary muscle function, decreased exercise tolerance and altered pulmonary immune response. Studies have shown that long-term (>1.0 years) nutritional rehabilitation results in either a slower rate of deterioration of pulmonary function or in its improvement especially if the patient is younger.

### EXAMPLE 4: Skin Application of the Colloidal Particles:

The mixed lipid formulation of Example 1 was additionally mixed with casein (1-2% casein by weight in the lipid mixture) or with visible or fluorescent dyes. Colloidal particles of mixtures with bicarbonate were made by the method described in Example 1. These colloidal particles were topically applied to the skin. Following this application, the colloid lipids that resided on the skin surface gave a desirable tactile sensibility, e.g., softness, and repelled wetting of the skin surface with water. The colloidal particles that contained the visible or fluorescent dyes were solubilized from the skin surface by detergents.

## Claims

1. A composition for sustained delivery of a bioactive agent to an individual comprising a mixture of colloidal particles in an aqueous environment, wherein each colloidal particle of the mixture comprises:
a. at least one non-esterified fatty acid having 8-24 carbon atoms;
b. at least one monoglyceride which is a monoester of glycerol and a fatty acid having 8-24 carbon atoms;
c. lysophosphatidylcholine in which the fatty acid moiety has 8-24 carbon atoms; and
d. a titrating agent;
wherein the fatty acids and monoglycerides together comprise from about 70.0 mole percent to about 99.0 mole percent of the lipid composition and the molar ratio of the fatty acids to the monoglycerides is from about 4:1 to about 1:4, and the lysophosphatidylcholine comprises from about 30.0 mole percent to about 1.0 mole percent of the lipid composition; wherein the lipid composition is in the form of colloidal particles in an aqueous environment; and wherein the mixture of colloidal particles is prepared from at least two separately prepared sets of colloidal particles wherein the average size distribution of the colloidal particles in a first set of colloidal particles and the average size distribution of colloidal particles in a second set of colloidal particles are different wherein at least 80% of the particle size distribution of the first set of colloidal particles is less than the average particle size of the second set of colloidal particles and at least 80% of the particle size distribution of the second set of colloidal particles is greater than the average particle size of the first set of colloidal particles.

2. The composition of claim 1 wherein the first set of colloidal particles has a first molar ratio of the titrating agent to the lysophosphatidylcholine, and the second set of colloidal particles has a second molar ratio of the titrating agent to the lysophosphatidylcholine, and wherein the first molar ratio of the first set of colloidal particles is less than 1.4:1 and the second molar ratio of the second set of colloidal particles is between greater than 1.4:1 and about 10:1, preferably, the second molar ratio is between 1.4:1 and about 7:1.

3. The composition of claims 1 or 2 wherein the titrating agent is bicarbonate.

4. The composition of claims 1, 2 or 3 wherein the fatty acids comprise omega-6 fatty acids and omega-3 fatty acids, and wherein a molar ratio of the omega-6 fatty acids to omega-3 fatty acids is about 5:1 and, preferably, the omega-6 fatty acids and the omega-3 fatty acids comprise at least 50% of the total fatty acids in the composition.

5. The composition of any of claims 1-4 further comprising at least one biologically compatible surfactant.

6. The composition of claim 5 wherein the second set of colloidal particles has a molar ratio of the titrating agent to the lysophosphatidylcholine of at least 7:1 and a molar ratio of the at least one biologically compatible surfactant to the lysophosphatidylcholine of at least 10:1.

7. The composition of claim 6 wherein the at least one biologically compatible surfactant is a bile salt, preferably sodium taurocholate.

8. The composition of any one of claims 1-7 wherein the bioactive agent is at least one drug.

9. The composition of any one of claims 1-8 wherein the bioactive agent is a polyunsaturated fatty acid and wherein at least one fatty acid moiety selected from the group consisting of the non-esterified fatty acids, the fatty acid moieties of the monoglycerides and/or the fatty acids of the lysophosphatidylcholine is polyunsaturated.

10. The composition of any one of claims 1-9 wherein the bioactive agent is fat and/or choline.

11. A pharmaceutical preparation comprising a composition as claimed in any one of claims 1-10 and a pharmaceutically acceptable carrier.

12. Use of a composition as claimed in any one of claims 1-10 for the manufacture of a medicament.

13. The use of claim 12 wherein the medicament is selected from the group consisting of an oral medicament, an inhalable medicament, and/or a topical medicament.

14. The use of claims 12 or 13 wherein the medicament is for treating a condition with a bioactive agent effective for treating the condition by administering to a subject in need of such treatment an amount of said composition effective for treating the composition.

15. The use of claims 12 or 13, wherein the medicament is for the delivery of a bioactive agent.

16. The use of claim 15 wherein the medicament is for treatment of the nutritional requirements of decreased brain choline, liver damage, pregnancy, lactation, total parenteral nutrition, and/or newborns.

17. The use of claim 15 wherein the medicament is for treatment of cystic fibrosis.

18. The use of a composition for the manufacture of a medicament for treatment of the nutritional requirements of decreased brain choline, liver damage, pregnancy, lactation, total parenteral nutrition, and/or newborns, the composition comprising:
a. at lease one non-esterified fatty acid having 8-24 carbon atoms;
b. at least one monoglyceride which is a monoester of glycerol and a fatty acid having 8-24 carbon atoms;
c. lysophosphatidylcholine in which the fatty acid moiety has 8-24 carbon atoms; and
d. a titrating agent,
wherein the fatty acids and monoglycerides together comprise from about 70.0 mole percent to about 99.0 mole percent of the lipid composition and the molar ratio of the fatty acids to the monoglycerides is from about 4:1 to about 1:4, and the lysophosphatidylcholine comprises from about 30.0 mole percent to about 1.0 mole percent of the lipid composition; wherein the lipid composition is in the form of colloidal particles in an aqueous environment; and wherein the mixture of colloidal particles is prepared from at least two separately prepared sets of colloidal particles wherein the average size distribution of the colloidal particles in a first set of colloidal particles and the average size distribution of colloidal particles in a second set of colloidal particles are different wherein at least 80% of the particle size distribution of the first set of colloidal particles is less than the average particle size of the second set of colloidal particles and at least 80% of the particle size distribution of the second set of colloidal particles is greater than the average particle size of the first set of colloidal particles.

19. The use of claim 18 wherein the concentration of the lysophosphatidylcholine is at least 0.1 mM in the aqueous environment and the molar ratio of the titrating agent to the lysophosphatidylcholine is greater than 1:1 in the aqueous environment, preferably greater than 1.4:1, and most preferably greater than 7:1.

20. The use of claims 18 or 19 wherein the titrating agent is bicarbonate.

21. The use of claim 20 wherein the composition further comprises a biologically compatible surfactant and wherein the molar ratio of the biologically compatible surfactant to the lysophosphatidylcholine in the aqueous environment is at least 10:1.

22. The use of claim 21 wherein the biologically compatible surfactant is a bile salt, preferably sodium taurocholate.

23. The use of a composition for the manufacture of a medicament for the treatment of cystic fibrosis, the composition comprising:
a. at least one non-esterified fatty acid having 8-24 carbon atoms;
b. at least one monoglyceride which is a monoester of glycerol and a fatty acid having 8-24 carbon atoms;
c. lysophosphatidylcholine in which the fatty acid moiety has 8-24 carbon atoms; and
d. a titrating agent,
wherein the fatty acids and monoglycerides together comprise from about 70.0 mole percent to about 99.0 mole percent of the lipid composition and the molar ratio of the fatty acids to the monoglycerides is from about 4:1 to about 1:4, and the lysophosphatidylcholine comprises from about 30.0 mole percent to about 1.0 mole percent of the lipid composition; wherein the lipid composition is in the form of colloidal particles in an aqueous environment; and wherein the mixture of colloidal particles is prepared from at least two separately prepared sets of colloidal particles wherein the average size distribution of the colloidal particles in a first set of colloidal particles and the average size distribution of colloidal particles in a second set of colloidal particles are different wherein at least 80% of the particle size distribution of the first set of colloidal particles is less than the average particle size of the second set of colloidal particles and at least 80% of the particle size distribution of the second set of colloidal particles is greater than the average particle size of the first set of colloidal particles.

24. A method for delivering a readily absorbable source of calories to an individual for non-therapeutic purposes, comprising the administration of one of the compositions claimed in claims 1-7, 9 and 10.

## Patentansprüche

1. Zusammensetzung zur anhaltenden Zuführung eines bioaktiven Mittels an einen Menschen, umfassend eine Mischung von Kolloidalen Partikeln in einer wäßrigen Umgebung, wobei jedes kolloidale Partikel der Mischung umfaßt:
a. mindestens eine nicht-veresterte Fettsäure mit 8-24 Kohlenstoffatomen;
b. mindestens ein Monoglycerid, das ein Monoester von Glycerol und einer Fettsäure mit 8-24 Kohlenstoffatomen ist;
c. Lysophosphatidylcholin, bei dem die Fettsäurekomponente 8-24 Kohlenstoffatome aufweist; und
d. ein Titrationsmittel,
wobei die Fettsäuren und Monoglyceride zusammen etwa 70,0 Molprozent bis etwa 99,0 Molprozent der Lipidzusammensetzung ausmachen und das Molverhältnis der Fettsäuren zu den Monoglyceriden etwa 4:1 bis etwa 1:4 beträgt und das Lysophosphatidylcholin etwa 30,0 Molprozent bis etwa 1,0 Molprozent der Lipidzusammensetzung ausmacht; wobei die Lipidzusammensetzung in der Form kolloidaler Partikel in einer wäßrigen Umgebung vorliegt;und wobei die Mischung von kolloidalen Partikeln zubereitet wird aus mindestens zwei getrennt zubereiteten Sätzen von kolloidalen Partikeln, wobei die durchschnittliche Größenverteilung der kolloidalen Partikel in einem ersten Satz von kolloidalen Partikeln und die durchschnittliche Größenverteilung der kolloidalen Partikel in einem zweiten Satz von kolloidalen Partikeln verschieden sind, wobei mindestens 80 % der Partikelgrößenverteilung des ersten Satzes von kolloidalen Partikeln kleiner ist als die durchschnittliche Partikelgröße des zweiten Satzes von kolloidalen Partikeln und mindestens 80 % der Partikelgrößenverteilung des zweiten Satzes von kolloidalen Partikeln größer ist als die durchschnittliche Partikelgröße des ersten Satzes von kolloidalen Partikeln.

2. Zusammensetzung nach Anspruch 1, wobei der erste satz von kolloidalen Partikeln ein erstes Molverhältnis des Titrationsmittels und der zweite Satz von kolloidalen Partikeln ein zweites Molverhältnis des Titrationsmittels zu dem Lysophosphatidylcholin aufweist, und wobei das erste Molverhältnis des ersten Satzes von kolloidalen Partikeln kleiner ist als 1,4:1 und das zweite Molverhältnis des zweiten Satzes von kolloidalen Partikeln größer als 1,4:1 bis etwa 10:1, bevorzugt zwischen 1,4:1 bis etwa 7:1, ist.

3. Zusammensetzung nach Anspruch 1 oder 2, wobei das Titrationsmittel Bicarbonat ist.

4. Zusammensetzung nach Anspruch 1, 2 oder 3, wobei die Fettsäuren Omega-6-Fettsäuren und Omega-3-Fettsäuren umfassen, und wobei ein Molverhältnis der Omega-6-Fettsauren zu Omega-3-Fettsäuren etwa 5:1 beträgt, und die Omega-6-Fettsäuren und die Omega-3-Fettsäuren bevorzugt mindestens 50 % der gesamten Fettsäuren in der Zusammensetzung umfassen.

5. Zusammensetzung nach einem der Ansprüche 1-4, umfassend mindestens ein biologisch kompatibles oberflächenaktives Mittel.

6. Zusammensetzung nach Anspruch 5, wobei der zweite Satz kolloidaler Partikel ein Molverhältnis des Titrationsmittels zu dem Lysophosphatidylcholin von mindestens 7:1 und ein Molverhältnis von dem mindestens einen biologisch kompatiblen oberflächenaktiven Mittel zu dem Lysophosphatidylcholin von mindestens 10:1 aufweist.

7. Zusammensetzung nach Anspruch 6, wobei das mindestens eine biologisch kompatible oberflächenaktive Mittel ein Gallensalz, bevorzugt Natrium-Taurocholat, ist.

8. Zusammensetzung nach einem der Ansprüche 1-7, wobei das bioaktive Mittel mindestens ein Arzneimittel ist.

9. Zusammensetzung nach einem der Ansprüche 1-8, wobei das bioaktive Mittel eine mehrfach ungesättigte Fettsäure ist und wobei mindestens eine Fettsäurekomponente, ausgewählt aus der Gruppe bestehend aus den nicht-veresterten Fettsäuren, den Fettsäurekomponenten der Monoglyceride und/oder den Fettsäuren des Lysophosphatidylcholins, mehrfach ungesättigt ist.

10. Zusammensetzung nach einem der Ansprüche 1-9, wobei das bioaktive Mittel Fett und/oder Cholin ist.

11. Eine pharmazeutische Zubereitung, die eine Zusammensetzung nach einem der Ansprüche 1-10 und einen pharmazeutisch akzeptablen Träger umfaßt.

12. Verwendung einer Zusammensetzung nach einem der Ansprüche 1-10 zur Herstellung eines Medikaments.

13. Verwendung nach Anspruch 12, wobei das Medikament ausgewählt ist aus der Gruppe bestehend aus einem oralen Medikament, einem inhalierbaren Medikament und/oder einem topischen Medikament.

14. Verwendung nach Anspruch 12 oder 13, wobei das Medikament zur Behandlung eines Zuscandes mit einem bei der Behandlung des Zustandes wirksamen bioaktiven Mittel durch Verabreichung einer Menge der bei der Behandlung des Zustandes wirksamen Zusammensetzung an eine Person, die dieser Behandlung bedarf, vorgesehen ist.

15. verwendung nach Anspruch 12 oder 13, wobei das Medikament für die Zuführung eines bioaktiven Mittels vorgesehen ist.

16. Verwendung nach Anspruch 15, wobei das Medikament für die Behandlung der Ernährungsbedürfnisse bei vermindertem Hirncholin, Leberschaden, Schwangerschaft, Laktation, vollständig parenteraler Ernährung und/oder Neugeborenen. vorgesehen ist.

17. Verwendung nach Anspruch 15, wobei das Medikament für die Behandlung von zystischer Fibrose vorgesehen ist.

18. vervendung einer Zusammensetzung zur Herstellung eines Medikaments zur Behandlung der Ernährungsbedürfnisse bei vermindertem Hirncholin, Leberschaden, Schwangerschaft, Laktation, vollständig parenteraler Ernährung und/oder Neugeborenen, wobei die Zusammensetzung umfaßt:
a. mindestens eine nicht-veresterte Fettsäure mit 8-24 Kohlenstoffatomen;
b. mindestens ein Monoglycerid, das ein Monoester von Glycerol und einer Fettsäure mit 8-24 Kohlenstoffatomen ist;
c. Lysophosphatidylcholin, bei dem die Fettsäurekomponente 8-24 Kohlenstoffatome aufweist; und
d. ein Titrationsmittel,
wobei die Fettsäuren und Monoglyceride zusammen etwa 70,0 Molprozent bis etwa 99,0 Molprozent der Lipidzusammensetzung ausmachen und das Molverhältnis der Fettsäuren zu den Monoglyceriden etwa 4:1 bis etwa 1:4 beträgt und das Lysophosphatidylcholin etwa 30,0 Molprozent bis etwa 1,0 Molprozent der Lipidzusammensetzung ausmacht; wobei die Lipidzusammensetzung in der Form kolloidaler Partikel in einer wäßrigen Umgebung vorliegt; und wobei die Mischung von kolloidalen Partikeln zubereitet wird aus mindestens zwei getrennt zubereiteten Sätzen von kolloidalen Partikeln, wobei die durchschnittliche Größenverteilung der kolloidalen Partikel in einem ersten Satz von kolloidalen Partikeln und die durchschnittliche Größenverteilung der kolloidalen Partikel in einem zweiten Satz von kolloidalen Partikeln verschieden sind, wobei mindestens 80 % der Partikelgrößenverteilung des ersten Satzes von kolloidalen Partikeln kleiner ist als die durchschnittliche Partikelgröße des zweiten Satzes von kolloidalen Partikeln und mindestens 80 % der Partikelgrößenverteilung des zweiten Satzes von kolloidalen Partikeln größer ist als die durchschnittliche Partikelgröße des ersten Satzes von kolloidalen Partikeln.

19. Verwendung nach Anspruch 18, wobei die Konzentration von dem Lysophosphatidylcholin in der wäßrigen Umgebung mindestens 0,1 mM beträgt und das Molverhältäis des Titrationsmittels zu dem Lysophosphatidylcholin in der wäßrigen Umgebung größer als 1:1, bevorzugt größer als 1,4:1 und am meisten bevorzugt größer als 7:1 ist.

20. Verwendung nach Anspruch 18 oder 19, wobei das Titrationsmittel Bicarbonat ist.

21. Verwendung nach Anspruch 20, wobei die Zusammensetzung zusätzlich ein biologisch kompatibles oberflächenaktives Mittel umfaßt und wobei das Molverhältnis des biologisch kompatiblen oberflächenaktiven Mittels zu dem Lysophosphatidylcholin in der wäßrigen Umgebung mindestens 10:1 beträgt.

22. Verwendung nach Anspruch 22, wobei das biologisch kompatible oberflächenaktive Mittel ein Gallensalz, bevorzugt Natrium-Taurocholat, ist.

23. Verwendung einer Zusammensetzung zur Herstellung eines Medikaments zur Behandlung von zystischer Fibrose, wobei die Zusammensetzung umfaßt:
a. mindestens eine nicht-veresterte Fettsäure mit 8-24 Kohlenstoffatomen;
b. mindestens ein Monoglycerid, das ein Monoester von Glycerol und einer Fettsäure mit 8-24 Kohlenstoffatomen ist;
c. Lysophosphatidylcholin, bei dem die Fettsäurekomponente 8-24 Kohlenstoffatome aufweist; und
d. ein Titrationsmittel;
wobei die Fettsäuren und Monoglyceride zusammen etwa 70,0 Molprozent bis etwa 99,0 Molprozent der Lipidzusammensetzung ausmachen und das Molverhältnis der Fettsäuren zu den Monoglyceriden etwa 4:1 bis etwa 1:4 beträgt und das Lysophosphatidylcholin etwa 30,0 Molprozent bis etwa 1,0 Molprozent der Lipidzusammensetzung ausmacht; wobei die Lipidzusammensetzung in der Form kolloidaler Partikel in einer wäßrigen Umgebung vorliegt; und wobei die Mischung von kolloidalen Partikeln zubereitet wird aus mindestens zwei getrennt zubereiteten Sätzen von kolloidalen Partikeln, wobei die durchschnittliche Größenverteilung der kolloidalen Partikel in einem ersten Satz von kolloidalen Partikeln und die durchschnittliche Größenverteilung der kolloidalen Partikel in einem zweiten Satz von kolloidalen Partikeln verschieden sind, wobei mindestens 80 % der Partikelgrößenverteilung des ersten Satzes von kolloidalen Partikeln kleiner ist als die durchschnittliche Partikelgröße des zweiten Satzes von kolloidalen Partikeln und mindestens 80 % der Partikelgrößenverteilung des zweiten Satzes von kolloidalen Partikeln größer ist als die durchschnittliche Partikelgröße des ersten Satzes von kolloidalen Partikeln.

24. Verfahren zum Zuführen einer leicht aufnehmbaren Quelle von Kalorien an ein Individuum für nichttherapeutische Zwecke, umfassend die Verabreichung von einer der Zusammensetzungen nach den Ansprüchen 1-7, 9 und 10.

## Revendications

1. Une composition pour la délivrance ou l'administration continue d'un agent bio-actif à un sujet, cette composition se composant d'un mélange de particules colloïdales dans un environnement aqueux, chaque particule colloïdale de ce mélange comprenant :
a. au moins un acide gras non estérifié à 8-24 atomes de carbone ;
b. au moins un monoglycéride consistant en un mono-ester de glycérol et un acide gras de 8-24 atomes de carbone ;
c. de la lysophosphatidylcholine, dans laquelle la portion acide gras comporte 8-24 atomes de carbone ; et
d. un agent de titrage,
composition dans laquelle les acides gras et les monoglycérides représentent ensemble depuis environ 70,0 mole pourcent jusqu'à environ 99,0 mole pourcent de la composition lipidique et le rapport molaire des acides gras par rapport aux monoglycérides est d'environ 4:1 à environ 1:4 et la lysophosphatidylcholine représente depuis environ 30,0 mole pourcent à environ 1,0 mole pourcent de la composition en lipides et dans laquelle, la composition des lipides se présente sous forme de particules colloïdales dans un environnement aqueux et dans laquelle le mélange de particules colloïdales est préparé à partir d'au moins deux séries préparées séparément de particules colloïdales dans lesquelles la répartition moyenne des tailles des particules colloïdales dans une première série de particules colloïdales et la répartition moyenne de taille des particules colloïdales dans une seconde séries de particules colloïdales sont différentes, dans la mesure où au moins 80% de la répartition des tailles de particules de la première série de particules colloïdales est inférieure à la taille moyenne de particules de la seconde série de particules colloïdales et au moins 80% de la répartition de tailles de particules de la seconde série de particules colloïdales est supérieure à la taille moyenne de particules de la première série de particules colloïdales.

2. La composition de la revendication 1 dans laquelle la première série de particules colloïdales présente un premier rapport moléculaire de l'agent de titrage par rapport à la lysophosphatidylcholine et la seconde série de particules colloïdales présente un second rapport moléculaire de l'agent de titrage par rapport à la lysophosphatidylcholine et dans laquelle le premier rapport molaire de la première série de particules colloïdales est inférieur à 1,4:1 et le second rapport molaire de la seconde série de particules colloïdales est compris entre plus de 1,4:1 et environ 10:1 et de préférence le second rapport molaire est compris entre 1,4:1 et environ 7:1.

3. La composition des revendications 1 ou 2 dans laquelle l'agent de titrage est un bicarbonate.

4. La composition selon les revendications 1, 2 ou 3 dans laquelle les acides gras consistent en acide gras 6-omega et en acide gras 3-omega, et dans laquelle un rapport molaire des acides gras 6-omega par rapport aux acides gras 3-omega est d'environ 5:1 et de préférence les acides gras 6-omega et les acides 3-omega représentent au moins 50% de la quantité totale d'acide gras de la composition.

5. La composition selon l'une quelconque des revendications 1-4, comprenant au moins un agent surfactif biologiquement compatible.

6. La composition de la revendication 5 dans laquelle la seconde série de particules colloïdales présente un rapport molaire de l'agent de titrage par rapport à la lysophosphatidylcholine d'au moins 7:1 et un rapport molaire du surfactif biologiquement compatible par rapport à la lysophosphatidylcholine d'au moins 10:1.

7. La composition de la revendication 6, dans laquelle le surfactif biologiquement compatible est un sel biliaire, de préférence du taurocholate de sodium.

8. La composition selon l'une quelconque des revendications 1-7, dans laquelle l'agent bioactif est au moins un médicament.

9. La composition selon l'une quelconque des revendications 1-8, dans laquelle l'agent bioactif est un acide gras poly-insaturé et dans laquelle au moins une portion acide gras sélectionnée dans le groupe constitué par les acides gras non-estérifiés, les portions acide gras des monoglycérides et/ou les acides gras de la lysophosphatidylcholine, est poly-insaturée.

10. La composition selon l'une quelconque des revendications 1-9, dans laquelle l'agent bioactif est une graisse et/ou de la choline.

11. Une préparation pharmaceutique comprenant une composition telle que revendiquée dans l'une quelconque des revendications 1-10 et un véhicule pharmaceutiquement acceptable.

12. Application d'une composition selon l'une quelconque des revendications 1-10 en vue de la préparation d'un médicament.

13. Application selon la revendication 12 dans laquelle le médicament est choisi dans le groupe constitué par un médicament administrable par voie orale, un médicament inhalable, et/ou un médicament administrable par voie topique.

14. L'application des revendications 12 ou 13 dans laquelle le médicament est destiné au traitement d'un état à un agent bioactif efficace pour le traitement de cet état par l'administration à un sujet nécessitant un tel traitement d'une quantité de ladite composition efficace pour le traitement de cet état.

15. L'application des revendications 12 ou 13 dans laquelle le médicament est destiné à l'administration d'un agent bioactif.

16. L'application de la revendication 15 dans laquelle le médicament est destiné au traitement de besoins nutritionnels correspondant à une diminution de la choline cervicale, une infection du foie, la grossesse, la lactation, la nutrition parentérale totale et/ou les nouveaux-nés.

17. L'application de la revendication 15 dans laquelle le médicament est destiné au traitement de la fibrose cystique.

18. L'application d'une composition en vue de la fabrication d'un médicament pour le traitement de besoins nutritionnels visant la diminution de la choline cervicale, les infections du foie, la grossesse, la lactation, la nutrition parentérale et/ou les nouveaux-nés, cette composition comprenant:
a. au moins un acide gras non estérifié ayant 8-24 atomes de carbone ;
b. au moins un monoglycéride consistant en un mono-ester de glycérol et d'un acide gras de 8-24 atomes de carbone ;
c. de la lysophosphatidylcholine, dans laquelle la portion acide gras comporte 8-24 atomes de carbone ; et
d. un agent de titrage,
composition dans laquelle les acides gras et les monoglycérides représentent ensemble entre environ 70,0 et environ 99,0 mole pourcent de la composition lipidique et le rapport molaire des acides gras par rapport aux monoglycérides est d'environ 4:1 à environ 1:4, et la lysophosphatidylcholine représente depuis environ 30,0 mole jusqu'à environ 1,0 mole pourcent de la composition lipidique, et dans laquelle la composition lipidique se présente sous forme de particules colloïdales dans un environnement aqueux, et dans laquelle le mélange de particules colloïdales est préparé à partir d'au moins deux séries préparées séparément de particules colloïdales, dans lesquelles la répartition moyenne des tailles des particules colloïdales dans une première série de particules colloïdales et la répartition moyenne de taille des particules colloïdales dans une seconde séries de particules colloïdales sont différentes, au moins 80% de la répartition de tailles de particules de la première série de particules colloïdales étant inférieure à la taille moyenne de particules de la seconde série de particules colloïdales et au moins 80% de la répartition de taille de particules de la seconde série de particules colloïdales est supérieure à la taille moyenne de particules de la première série de particules colloïdales.

19. L'application de la revendication 18, dans laquelle la concentration de la lysophosphatidylcholine est d'au moins 0,1 mM dans l'environnement aqueux et le rapport molaire de l'agent de titrage par rapport à la lysophosphatidylcholine est supérieur à 1,1 dans l'environnement aqueux et de préférence supérieur à 1,4:1 et plus préférablement supérieur à 7:1.

20. L'application des revendications 18 ou 19, dans laquelle l'agent de titrage est un bicarbonate.

21. L'application de la revendication 20 dans laquelle la composition comporte au surplus un surfactif biologiquement compatible et dans laquelle le rapport molaire du surfactif biologiquement compatible par rapport à la lysophosphatidylcholine dans l'environnement aqueux est d'au moins 10:1.

22. L'application de la revendication 21 dans laquelle le surfactif biologiquement compatible est un sel biliaire, de préférence du taurocholate de sodium.

23. L'application d'une composition destinée à la fabrication d'un médicament pour le traitement de la fibrose cystique, cette composition comprenant :
a. au moins un acide gras non estérifié ayant 8-24 atomes de carbone ;
b. au moins un monoglycéride consistant en un mono-ester de glycérol et d'un acide gras de 8-24 atomes de carbone ;
c. de la lysophosphatidylcholine, dans laquelle la portion acide gras comporte 8-24 atomes de carbone ; et
d. un agent de titrage,
application dans laquelle les acides gras et les monoglycérides représentent ensemble depuis environ 70,0 jusqu'à environ 99,0 mole pourcent de la composition lipidique et le rapport molaire des acides gras par rapport aux monoglycérides est d'environ 4:1 à environ 1:4, et la lysophosphatidylcholine représente depuis environ 30,0 jusqu'à environ 1,0 mole pourcent de la composition lipidique, composition dans laquelle la composition lipidique est sous forme de particules colloïdales dans un environnement aqueux, et dans laquelle le mélange de particules colloïdales est préparé à partir d'au moins deux séries préparées séparément de particules colloïdales, dans lesquelles la répartition moyenne de tailles des particules colloïdales dans une première série de particules colloïdales et la répartition moyenne de taille des particules colloïdales dans une seconde séries de particules colloïdales sont différentes, au moins 80% de la répartition de tailles de particules de la première série de particules colloïdales est inférieure à la taille moyenne de particules de la seconde série de particules colloïdales et au moins 80% de la répartition de taille de particules de la seconde série de particules colloïdales est supérieure à la taille moyenne de particules de la première série de particules colloïdales.

24. Un procédé pour l'administration d'une source facilement absorbable de calories à un individu, à des fins non thérapeutiques, consistant à administrer l'une des compositions selon l'une des revendication 1-7, 9 et 10.
